# EUROPEAN PATENT SPECIFICATION

(11) **EP 1 853 305 B1**
(45) Date of publication and mention of the grant of the patent: **20.08.2014**
(21) Application number: 06704603.7
(22) Date of filing: 03.02.2006
(51) Int. Cl.: A61K 39/00, A61K 38/04, C07K 14/47

(54) **SURVIVIN PEPTIDE VACCINE**
SURVIVIN-PEPTID-VAKZIN
VACCIN A BASE DE PEPTIDE DE SURVIVINE

(30) Priority: 04.02.2005 DK 200500173; 07.02.2005 US 650751 P
(43) Date of publication of application: 14.11.2007
(60) Divisional application: 10184070.0
(73) Proprietor: Survac ApS, 1826 Frederiksberg (DK)
(72) Inventor: ANDERSEN, Mads Hald, 2850 Nærum (DK)
(74) Representative: Høiberg A/S
(86) International application number: PCT/DK2006/000061
(87) International publication number: WO 2006/081826

(56) References cited:
- WO-A-2004/022709
- WO-A-2004/067023
- WO-A-2004/067023
- WO-A-2005/049073
- WO-A-2005/049073
- JP-A- 2002 284 797
- REKER SINE ET AL: "Identification of novel survivin-derived CTL epitopes." CANCER BIOLOGY & THERAPY. FEB 2004, vol. 3, no. 2, February 2004 (2004-02), pages 173-179, XP002391401 ISSN: 1538-4047
- F.R. VOGEL ET AL.: "A compendium of vaccine adjuvants and excipients" INTERNET ARTICLE, [Online] XP002391402 Retrieved from the Internet: URL:HTTP://WWW.NIAID.NIH.GOV/DAIDS/VACCINE /PDF/COMPENDIUM.PDF> [retrieved on 2002]
- MACHIELS JEAN-PASCAL ET AL: "Peptide-based cancer vaccines" SEMINARS IN ONCOLOGY, vol. 29, no. 5, October 2002 (2002-10), pages 494-502, XP009069719 ISSN: 0093-7754
- WANG F ET AL: "Phase I trial of a MART-1 peptide vaccine with incomplete Freund's adjuvant for resected high-risk melanoma" CLINICAL CANCER RESEARCH, THE AMERICAN ASSOCIATION FOR CANCER RESEARCH, US, vol. 5, no. 10, October 1999 (1999-10), pages 2756-2765, XP002235402 ISSN: 1078-0432
- NOGUCHI M ET AL: "Phase I trial of patient-oriented vaccination in HLA-A2-positive patients with metastatic hormone-refractory prostate cancer" CANCER SCIENCE, JAPANESE CANCER ASSOCIATION, TOKYO, JP, vol. 95, no. 1, January 2004 (2004-01), pages 77-84, XP002996950 ISSN: 1347-9032
- YAMSHCHIKOV G V ET AL: "Evaluation of peptide vaccine immunogenicity in draining lymph nodes and peripheral blood of melanoma patients" INTERNATIONAL JOURNAL OF CANCER, NEW YORK, NY, US, vol. 92, no. 5, 1 June 2001 (2001-06-01), pages 703-711, XP002331594 ISSN: 0020-7136
- DRIEL VAN W J ET AL: "VACCINATION WITH HPV16 PEPTIDES OF PATIENTS WITH ADVANCED CERVICAL CARCINOMA: CLINICAL EVALUATION OF A PHASE I-II TRIAL" EUROPEAN JOURNAL OF CANCER, PERGAMON PRESS, OXFORD, GB, vol. 35, no. 6, June 1999 (1999-06), pages 946-952, XP000986010 ISSN: 0959-8049
- TALEBI TONY ET AL: "Peptide vaccine trials for melanoma: Preclinical background and clinical results." SEMINARS IN CANCER BIOLOGY, vol. 13, no. 6, December 2003 (2003-12), pages 431-438, XP002391403 ISSN: 1044-579X
- TSUBOI AKIHIRO ET AL: "WT1 peptide-based immunotherapy for patients with lung cancer: Report of two cases." MICROBIOLOGY AND IMMUNOLOGY, vol. 48, no. 3, 2004, pages 175-184, XP002391404 ISSN: 0385-5600
- SCALZO ANTHONY A ET AL: "Induction of protective cytotoxic T cells to murine cytomegalovirus by using a nonapeptide and a human-compatible adjuvant (Montanide ISA 720)" JOURNAL OF VIROLOGY, vol. 69, no. 2, 1995, pages 1306-1309, XP002391405 ISSN: 0022-538X
- OKA YOSHIHIRO ET AL: "Induction of WT1 (Wilms' tumor gene)-specific cytotoxic T lymphocytes by WT1 peptide vaccine and the resultant cancer regression" PROCEEDINGS OF THE NATIONAL ACADEMY OF SCIENCES OF THE UNITED STATES OF AMERICA, vol. 101, no. 38, 21 September 2004 (2004-09-21), pages 13885-13890, XP002391406 ISSN: 0027-8424
- ANDERSEN M H ET AL: "Identification of a cytotoxic T lymphocyte response to the apoptosis inhibitor protein survivin in cancer patients" CANCER RESEARCH, AMERICAN ASSOCIATION FOR CANCER RESEARCH, BALTIMORE, MD, US, vol. 61, no. 3, 1 February 2001 (2001-02-01), pages 869-872, XP002283853 ISSN: 0008-5472
- REKER SINE ET AL: "HLA-B35-restricted immune responses against survivin in cancer patients." INTERNATIONAL JOURNAL OF CANCER, vol. 108, no. 6, 1 March 2004 (2004-03-01), pages 937-941, XP002465840 ISSN: 0020-7136
- HIROHASHI Y ET AL: "An HLA-A24-restricted cytotoxic T lymphocyte epitope of a tumor-associated protein, survivin" CLINICAL CANCER RESEARCH, THE AMERICAN ASSOCIATION FOR CANCER RESEARCH, US, vol. 8, no. 6, June 2002 (2002-06), pages 1731-1739, XP002283856 ISSN: 1078-0432
- TSURUMA TETSUHIRO ET AL: "Phase I clinical study of anti-apoptosis protein, survivin-derived peptide vaccine therapy for patients with advanced or recurrent colorectal cancer" JOURNAL OF TRANSLATIONAL MEDICINE, BIOMED CENTRAL, LONDON, GB, vol. 2, no. 1, 13 June 2004 (2004-06-13), page 19, XP021009821 ISSN: 1479-5876
- REKER SINE ET AL: "Identification of novel survivin-derived CTL epitopes." CANCER BIOLOGY & THERAPY. FEB 2004, vol. 3, no. 2, February 2004 (2004-02), pages 173-179, XP002391401 ISSN: 1538-4047
- F.R. VOGEL ET AL.: "A compendium of vaccine adjuvants and excipients" INTERNET ARTICLE, [Online] XP002391402 Retrieved from the Internet: URL:HTTP://WWW.NIAID.NIH.GOV/DAIDS/VACCINE /PDF/COMPENDIUM.PDF> [retrieved on 2002]
- MACHIELS JEAN-PASCAL ET AL: "Peptide-based cancer vaccines" SEMINARS IN ONCOLOGY, vol. 29, no. 5, October 2002 (2002-10), pages 494-502, XP009069719 ISSN: 0093-7754
- WANG F ET AL: "Phase I trial of a MART-1 peptide vaccine with incomplete Freund's adjuvant for resected high-risk melanoma" CLINICAL CANCER RESEARCH, THE AMERICAN ASSOCIATION FOR CANCER RESEARCH, US, vol. 5, no. 10, October 1999 (1999-10), pages 2756-2765, XP002235402 ISSN: 1078-0432
- NOGUCHI M ET AL: "Phase I trial of patient-oriented vaccination in HLA-A2-positive patients with metastatic hormone-refractory prostate cancer" CANCER SCIENCE, JAPANESE CANCER ASSOCIATION, TOKYO, JP, vol. 95, no. 1, January 2004 (2004-01), pages 77-84, XP002996950 ISSN: 1347-9032
- YAMSHCHIKOV G V ET AL: "Evaluation of peptide vaccine immunogenicity in draining lymph nodes and peripheral blood of melanoma patients" INTERNATIONAL JOURNAL OF CANCER, NEW YORK, NY, US, vol. 92, no. 5, 1 June 2001 (2001-06-01), pages 703-711, XP002331594 ISSN: 0020-7136
- DRIEL VAN W J ET AL: "VACCINATION WITH HPV16 PEPTIDES OF PATIENTS WITH ADVANCED CERVICAL CARCINOMA: CLINICAL EVALUATION OF A PHASE I-II TRIAL" EUROPEAN JOURNAL OF CANCER, PERGAMON PRESS, OXFORD, GB, vol. 35, no. 6, June 1999 (1999-06), pages 946-952, XP000986010 ISSN: 0959-8049
- TALEBI TONY ET AL: "Peptide vaccine trials for melanoma: Preclinical background and clinical results." SEMINARS IN CANCER BIOLOGY, vol. 13, no. 6, December 2003 (2003-12), pages 431-438, XP002391403 ISSN: 1044-579X
- TSUBOI AKIHIRO ET AL: "WT1 peptide-based immunotherapy for patients with lung cancer: Report of two cases." MICROBIOLOGY AND IMMUNOLOGY, vol. 48, no. 3, 2004, pages 175-184, XP002391404 ISSN: 0385-5600
- SCALZO ANTHONY A ET AL: "Induction of protective cytotoxic T cells to murine cytomegalovirus by using a nonapeptide and a human-compatible adjuvant (Montanide ISA 720)" JOURNAL OF VIROLOGY, vol. 69, no. 2, 1995, pages 1306-1309, XP002391405 ISSN: 0022-538X
- OKA YOSHIHIRO ET AL: "Induction of WT1 (Wilms' tumor gene)-specific cytotoxic T lymphocytes by WT1 peptide vaccine and the resultant cancer regression" PROCEEDINGS OF THE NATIONAL ACADEMY OF SCIENCES OF THE UNITED STATES OF AMERICA, vol. 101, no. 38, 21 September 2004 (2004-09-21), pages 13885-13890, XP002391406 ISSN: 0027-8424

## Description

### Field of the Invention

The present invention relates to a therapeutic vaccine comprising one or more survivin polypeptide fragments. The vaccine can be used for prophylactic, ameliorating and/or curative treatment of e.g. cancer diseases. The specification further describes methods of combination treatment.

### Background of invention

The mammalian immune system recognizes and reacts to foreign or alien materials. An important facet of the system is the T-cell response. This response requires that T cells recognize and interact with complexes of cell surface molecules, referred to as human leukocyte antigens (HLA) constituting the human major histocompatibility complex (MHC), and peptides. The peptides are derived from larger molecules, which are processed by the cells, which also present the HLA/MHC molecule. The Interaction of T cells and completes of HLA/peptide is restricted, requiring a T cell that Is specific for a particular combination of an HLA molecule and a peptide, If a specific T cell is not present, there Is no T-cell response even if its partner complex is present Similarly, there is no response if the specific complex is absent, but the T cell Is present.

It is well established that peptide epitopes derived from human tumor-associated antigens (TAA) can be recognized by cytotoxic T lymphocytes (CTL) in the context of MHC molecules and that most, if not all, tumors express such antigens. Consequently, exciting clinical efforts are ongoing to target these TAA in strategies such as vaccination and adoptive T cell therapy in order to generate effective anti-tumor CTL responses in patients.

However, immunoselection of antigen loss variants can be a serious obstacle for the curative potential of most of the known CTL epitopes in clinical oncology, and the selection of antigen deficient mutant tumors Is a well-recognized limitation in therapeutic strategies when targeting antigens that do not have a role in cancer growth.

The reason is that most characterized peptides are derived from polypeptides, which are not essential for the survival of the tumor cell. Thus, if powerful CTL responses are induced against these peptide antigens by therapeutical measures such as vaccinations, tumor cells lacking the expression of the targeted antigen are very likely to escape the raised immune responses.

There is a need for more efficient therapeutic vaccines and improved methods of treatment of cancer diseases.

The mechanism by which T cells recognize cellular abnormalities has also been implicated in cancer. In WO92/20356, a family of genes is disclosed which are processed into peptides which, in turn, are expressed on cells surfaces, and can lead to lysis of the tumor cells by specific CTLs. These genes are referred to as the MAGE family and are said to code for "tumor rejection antigen precursors" or "TRAP" molecules, and the peptides derived there from are referred to as "tumor rejection antigens" or "TRAs".

However, although it is generally accepted that most if not all, tumors are antigenic, only a few are indeed immunogenic in the sense that tumor progression is readily controlled by the immune system.

To overcome this, several immunotherapeutic trials have been initiated, using vaccinations with TAA-derived peptides. For melanoma, the tumor for which the largest number of CTL-defined TAAs has been characterized, powerful CTL responses against antigens have been induced by vaccination and some patients experienced a complete remission of their disease. However, most of the peptide epitopes used in these vaccination trials are melanocyte specific, and these peptides cannot be applied for tumors of non-melanocyte origin. Furthermore, expression of these TAAs is heterogeneous among tumors from different patients and can even vary among metastases obtained from one patient. However, during the last couple of years a number of tumor specific peptide antigens, which are expressed in a number of different cancers, have been identified, i. e. HER-2, Muc-1 and telomerase.

It has also been shown that by proper manipulation tumor antigens present in tumors can be exposed to the immune system. Studies have shown that the CD8+ CTL arm of can be exposed to the immune system. Studies have shown that the CD8+ CTL arm of the immune response, alone or in combination with CD4+ Th cells, constitutes the primary anti-tumor effector arm of the adaptive immune response. Up till now the focus has mainly been on the CTL arm of the immune response. However, it is becoming clearer that the CD4 T cell response plays an essential role in tumor rejection, especially in the induction phase or in the extension of a CTL response in vivo. Consequently, the incorporation of class 1-restricted tumor antigens into effective tumor vaccination protocols might increase the effectiveness of the vaccines.

Apoptosis is a genetic program of cellular suicide, and inhibition of apoptosis has been suggested to be an important mechanism involved in cancer formation by extending the life span of cells favouring the accumulation of transforming mutations. Survivin is a recently identified member of the family of inhibitors of apoptosis proteins (IAPs). In a global gene expression analysis of about 4 million transcripts, survivin was identified as one of the top genes invariably up-regulated in many types of cancer but not in normal tissue. Solid malignancies overexpressing survivin include lung, colon, breast, pancreas, and prostate cancer as well as hematopoietic malignancies. Additionally, series of melanoma and non-melanoma skin cancers have been reported to be invariably survivin positive. The overexpression of survivin in most human cancers suggests a general role of apoptosis inhibition in tumor progression, a notion substantiated by the observation that in the case of colorectal and bladder cancer, as well as neuroblastoma, expression of survivin was associated with an unfavourable prognosis. Survivin is as other apoptosis inhibitors expressed in the endothelia cells during tumor-angiogenesis, and anti-sense targeting of survivin during angiogenesis causes endothelial-cell apoptosis, which promotes rapid collapse of the capillary -like vessels *in vitro.* In contrast, survivin is undetectable in normal adult tissues. As survivin is overexpressed in most human cancers and inhibition of its function results in increased apoptosis, this protein could be a target for therapeutic CTL responses. The survivin protein and the potential diagnostic and therapeutic use hereof are disclosed in US 6,245,523. Survivin is a 16. 5 kDa cytoplasmic protein containing a single BIR and a highly charged carboxy-terminal coiled region instead of a RING finger, which inhibits apoptosis induced by growth factor (IL-3) withdrawal when transferred in B cell precursors. The gene coding for survivin is nearly identical to the sequence of Effector Cell Protease Receptor-1 (EPR-1), but oriented in the opposite direction, thus suggesting the existence of two separate genes duplicated in a head-to head configuration. Accordingly, survivin can be described as an antisense EPR-1 product. Due to the opposite orientation of the two genes the amino acid sequences of the encoded in proteins are different.

Functionally, inhibition of survivin expression by up-regulating its natural antisense EPR-1 transcript results in massive apoptosis and decreased cell growth.

US 6,245.523 discloses the isolation of purified survivin and it provides nucleic acid molecules that encode the survivin protein, and antibodies and other molecules that bind to survivin. US 6,245,523 also discloses anti-apoptotically active fragments of the survivin protein and variants hereof wherein an amino acid residue has been inserted N-or C-terminal to, or within, the disclosed survivin sequence. It is specifically disclosed that such peptides should contain key functional residues required for apoptosis, i. e. Trp at position 67, Pro at position 73 and Cys at position 84.

It has previously been disclosed that a weak specific T-cell response may be raised using vaccination with dendritic cells loaded with survivin peptides as measured by ELISPOT assay (WO2004/067023 and Otto, K. et al., J. Vaccine (2004)). The response was less than 30 per 10⁴ cells and the following evaluation of the clinical result showed progressive disease. Therefore this vaccine has only very limited usage. Thus, it is of great interest to develop vaccines capable of inducing a very strong specific T-cell response and particularly capable of inducing a clinical response wherein the progression of the disease is inhibited.

### Summary of invention

The present invention is based on the discovery that MHC Class I restricted peptides derived from the survivin protein, are capable of binding to MHC Class I HLA molecules and thereby eliciting both ex vivo and in situ CTL immune responses in patients suffering from a wide range of cancer diseases. Prior to use in treatment it is essential to analyze the immune responses in combination with clinical results, to evaluate the usefulness of a vaccine composition for the treatment of cancer. A specific T-cell response after immunization may be a useful test for potential antigens useful in vaccines, it is preferred that the vaccine composition is capable of eliciting a very strong T-cell response, such as more than 50 INF-γ releasing cells per 10⁴ PBMCs, as this may increase the success of the vaccine composition for use in treatment of cancer. The present invention discloses particular effective vaccine compositions capable of inducing partial or complete tumor regression. Evidently, these findings open the way for novel therapeutic approaches which, due to the fact that survivin appears to be expressed universally by tumor cells, are generally applicable in the control of cancer diseases.

A vaccine composition or immunogenic composition as described herein qualifies as a pharmaceutical composition as the peptides give rise to a CTL response capable of combating neoplasia. Therefore the vaccine composition qualifies as a therapeutic vaccine or a therapeutic or pharmaceutical composition.

The present invention relates to a vaccine composition comprising,
i. one or more HLA-A2 restricted survivin peptide selected from the group consisting of LLLGEFLKL (SEQ ID NO. 4) and LMLGEFLKL (SEQ ID NO 5) or peptide variants consisting of 5-20 amino acid residues, wherein the sequence of the peptide variant, over the entire length, is at least 85 % identical to a consecutive amino acid sequence of SEQ ID NO: 23, and
ii. an adjuvant formulated for a water in oil emulsion comprising a mineral oil and a surfactant, wherein the adjuvant comprises up to 14,5 % Vol. of said surfactant for use as a medicament.

In an aspect the specification relates to a vaccine composition comprising, one or more survivin peptides or survivin peptide variants, wherein the sequence of the peptide variant, over the entire length, is at least 85 % identical to a consecutive amino acid sequence of SEQ ID NO: 23, and an adjuvant formulated for a water in oil emulsion comprising a mineral oil and a surfactant, wherein the adjuvant comprises up to 14,5 % Vol. of said surfactant, for use as a medicament.

An aspect of the specification relates to a survivin peptide variant of at the most 50 amino acid residues capable of binding HLA-B7 comprising a peptide selected from the group of: APPAWQPFL (SEQ ID NO: 13) and RPPAWQPFL (SEQ ID NO: 14).

The specification also describes vaccine compositions comprising one or more survivin peptide or peptide variants, wherein the sequence of the peptide variant, over the entire length, is at least 85 % identical to a consecutive amino acid sequence of SEQ ID NO: 23, and wherein the composition comprises:
i. a HLA-B7 binding peptide and/or
   a HLA-A1 and a HLA-A2 restricted peptide and/or
   a HLA-A1 and a HLA-B35 restricted peptide
ii. and an adjuvant.

The specification describes a vaccine composition comprising, three or more survivin peptide or survivin peptide variants, wherein the sequence of the peptide variant, over the entire length, is at least 85 % identical to a consecutive amino acid sequence of SEQ ID NO: 23 and wherein,
i. at least one peptide or peptide variant Is selected from the group of HLA-A1 binding peptides, and wherein
ii. at least one peptide or peptide variant is selected from the group of HLA-A2 binding peptides, and wherein,
iii. at least one peptide or peptide variant is selected from the group of HLA-B35
binding peptides,

### and an adjuvant.

### The specification also describes

a vaccine composition comprising seven or more survivin peptide or survivin peptide variants, wherein the sequence of the peptide variant, over the entire length, Is at least 85 % Identical to a consecutive amino acid sequence of SEQ ID NO: 23,
i. and wherein at least one peptide or peptide variant is selected from the group of HLA-A1 binding peptides,
ii. and wherein at least one peptide or peptide variant is selected from the group of HLA-A2 binding peptides,
iii. and wherein at least one peptide or peptide variant is selected from the group of HLA-A3 binding peptides
iv. and wherein at least one peptide or peptide variant is selected from the group of HLA-A24 binding peptides
v. and wherein at least one peptide or peptide variant is selected from the group of HLA-A11 binding peptides
vi. and wherein at least one peptide or peptide variant is selected from the group of HLA-B35 binding peptides
vii. and wherein at least one peptide or peptide variant is selected from the group of HLA-B7 binding peptides
and an adjuvant.

Development of solid tumors is dependent on blood vessels formation. Inhibition of angiogenesis may thereby prevent development of solid tumors. Expression of survivin, bcl-2 and Mcl-1 in the endothelia cells during tumor-angiogenesis, suggest that vaccinations using peptides as described herein may have an anti-angiogenic effect.

The specification also describes a vaccine comprising one or more survivin peptide or peptide variants, wherein the sequence of the peptide variant, over the entire length, is at least 85 % identical to a consecutive amino acid sequence of SEQ ID NO: 23, and an adjuvant, capable of inducing infiltration of antigen specific t-cells in tumor stroma in a subject for use as a medicament

In one further aspect the specification relates to a vaccine composition comprising:
i. a nucleic acid encoding:
   a) the survivin polypeptide (SEQ ID NO: 23),
   b) a survivin peptide or
   c) a survivin peptide variant, wherein the sequence of the peptide variant, over the entire length, is at least 85 % identical to a consecutive amino add sequence of SEQ ID NO: 23, and
ii. an adjuvant.

An aspect of the invention relates to the use of a vaccine composition comprising one or more survivin peptide or survivin peptide variants and an adjuvant, capable of eliciting a very strong specific cytotoxic T- cell response in a subject for the manufacture of a medicament. This is relevant as a very strong specific T- cell response can be correlated with a higher rate of good clinical responses. In this regard a very strong specific T-cell response equals a response of more than 50 peptide specific spots per 10⁴ PBMC cells, when measured by ELISPOT assay, before and after administration of the vaccine composition,

An aspect of the specification relates to a kit in parts comprising;
i. a vaccine composition comprising;
   a) one or more survivin peptide or survivin peptide variants, wherein the sequence of the peptide variant, over the entire length, is at least 85 % identical to a consecutive amino acid sequence of SEQ ID NO: 23,
   b) and an adjuvant, and
ii. a secondary medicament.

An aspect of the invention describes a method of stimulating a strong specific T-cell response against survivin In a subject, said method comprising:
a) providing a vaccine composition according to the invention,
b) administering said vaccine composition to the subject, wherein said vaccine composition may be administered more than once; and
c) thereby stimulating a strong specific T-cell response in the subject, wherein the strong specific T-cell response, when measured by ELISPOT assay, before and after administration of the vaccine composition, is more than 50 peptide specific spots per 10⁴ PBMC cells,
d) obtaining a strong specific T-cell response in the subject

A further aspect of the invention relates to a method of treatment or preventing a disease comprising;
a) providing a vaccine composition according to the invention,
b) administering said vaccine composition to a subject, wherein said vaccine composition is administered more than once.

In an additional aspect the invention relates to a method of treatment or preventing a disease comprising;
a) providing a vaccine composition according to the Invention,
b) administering said vaccine composition to a subject, wherein said vaccine composition is administered more than once.
c) thereby stimulating a strong specific T-cell response in the subject, wherein the specific T-cell response, when measured by ELISPOT assay, before and after administration of the vaccine composition, is more than 50 peptide specific spots per 10⁴ PBMC cells,
d) obtaining a clinical response in the subject.

In certain aspects the specification relates to methods of inducing infiltration of antigen specific T- cells in tumor stroma in a subject or inhibiting angiogenesis in a subject comprising;
a) providing a vaccine according to the Invention,
b) administering said vaccine composition to a subject,
c) obtaining infiltration of antigen specific T- cells in tumor stroma or inhibition of angiogenesis.

A method of combination therapy including simultaneously, sequentially or separate administration in any order, of:
a) a vaccine composition according to the invention and,
b) a secondary medicament.

### Definitions:

### AA: See "Amino acid".

**Adjuvant:** A vaccine adjuvant is a component that increases the specific immune response to an antigen.

**Amino acid:** Entity comprising an amino terminal part (NH₂) and a carboxy terminal part (COOH) separated by a central part comprising a carbon atom, or a chain of carbon atoms, comprising at least one side chain or functional group. NH₂ refers to the amino group present at the amino terminal end of an amino acid or peptide, and COOH refers to the carboxy group present at the carboxy terminal end of an amino acid or peptide. The generic term amino acid comprises both natural and non-natural amino acids. Natural amino acids of standard nomenclature as listed in J. Biol. Chem., 243: 3552-59 (1969) and adopted in 37 C.F.R., section 1.822(b)(2) belong to the group of amino acids listed in Table 1 herein below. Non-natural amino acids are those not listed in Table 1. Examples of non-natural amino acids are those listed e.g. in 37 C.F.R. section 1.822(b) (4), all of which are incorporated herein by reference. Amino acid residues described herein can be in the "D" or "L" isomeric form.

| Symbols | | Amino acid |
|---|---|---|
| 1-Letter | 3-Letter | |
| Y | Tyr | tyrosine |
| G | Gly | glycine |
| F | Phe | phenylalanine |
| M | Met | methionine |
| A | Ala | alanine |
| S | Ser | serine |
| I | Ile | isoleucine |
| L | Leu | leucine |
| T | Thr | threonine |
| V | Val | valine |
| P | Pro | proline |

**Table 1. Natural amino acids and their respective codes.**

| | | |
|---|---|---|
| K | Lys | lysine |
| H | His | histidine |
| Q | Gln | glutamine |
| E | Glu | glutamic acid |
| W | Trp | tryptophan |
| R | Arg | arginine |
| D | Asp | aspartic acid |
| N | Asn | asparagine |
| C | Cys | cysteine |

**Amino acid residue:** the term "amino acid residue" is meant to encompass amino acids, either standard amino acids, non-standard amino acids or pseudo-amino acids, which have been reacted with at least one other species, such as 2, for example 3, such as more than 3 other species. In particular amino acid residues may comprise an acyl bond in place of a free carboxyl group and/or an amine-bond and/or amide bond in place of a free amine group. Furthermore, reacted amino acid residues may comprise an ester or thioester bond in place of an amide bond.

**Antibody:** Are immunoglobulin molecules and active portions of immunoglobulinmolecules. Antibodies are for example intact immunoglobulin molecules or fragments thereof retaining the immunologic activity.

**Antigen:** The molecule recognised by an antibody. Usually a peptide, polypeptide or a multimeric polypeptide. Antigens are preferably capable of eliciting an immune response.

**A.P.I. gravity:** A term used by the petroleum industry to express the relative density of petroleum liquids according to values determined by the API (American Petroleum Institute). API gravity is measured by a hydrometer instrument having a scale graduated in degrees API.

**CTL:** Cytotoxic T lymphocytes. A sub group of T-cells expressing CD8 along with the T-cell receptor and therefore able to respond to antigens presented by class I molecules.

**Emulsion:** A suspension of small globules of one liquid in a second liquid with which the first will not mix.

**Emulsifier:** a surface-active agent that promotes the formation of an emulsion

**HLA:** Human leukocute antigen also named MHC. Three different MHC class I molecules, HLA-A, HLA-B and HLA-C are synthesised by humans. Human MHC class II molecules are designated HLA-D.

**Immunoglobulin:** The serum antibodies, including IgG, IgM, IgA, IgE and IgD **Isolated:** is used to describe any of the various secretagogues, polypeptides and nucleotides disclosed herein, that have been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified.

**Ligand:** A molecule, for example a peptide, capable of specific binding to one or more cognate receptors. An antigen is, for example, a ligand to its cognate antibodies. **MHC:** Major histocompatability complex also named HLA. Two main subclasses of MHC, Class I and Class II exists.

**Mineral oil:** Oil derived from a mineral source, such as petroleum, as opposed to oils derived from plants and animals. Hydrocarbon mixtures of varying compositions.

**Montanide ISA** (Montanide Incomplete Seppic Adjuvant): An oily adjuvant formulated for a water in oil emulsion. Montanide ISA adjuvants are a group of oil/surfactant-based adjuvants where a non-metabolizable and/or metabolizable oil is combined with surfactants (available from Seppic, Belgium).

**PBMC:** Peripheral blood mononuclear cell.

**PBL:** Peripheral blood leukocyte.

**Peptide:** Plurality of covalently linked amino acid residues defining a sequence and linked by amide bonds. The term is used analogously with oligopeptide and polypeptide. The natural and/or non-natural amino acids may be linked by peptide bonds or by non-peptide bonds. The term peptide also embraces post-translational modifications introduced by chemical or enzyme-catalyzed reactions, as are known in the art.

**Surface tension:** Surface tension is the energy required to increase the surface area. The cohesive forces between liquid molecules are responsible for the surface tension and the molecules at a liquid surface cohere strongly to each other. **Surfactant:** A surface active agents capable of reducing the surface tension of a liquid in which it is dissolved. A surfactant is a compound containing a polar group which is hydrophilic and a non polar group which is hydrophobic and often composed of a fatty chain.

**TAA:** Tumor -associated antigen

### Detailed description of the invention

The present invention regards vaccine compositions comprising survivin peptides and survivin peptide variants as describe here below.

### Vaccine compositions

Vaccine composition according to the invention can be formulated according to known methods such as by the admixture of one or more pharmaceutically acceptable excipients or carriers with the active agent, preferably acceptable for administration to humans. Examples of such excipients, carriers and methods of formulation may be found e.g. in Remington's Pharmaceutical Sciences (Maack Publishing Co, Easton, PA). To formulate a pharmaceutically acceptable composition suitable for effective administration, such compositions will according to the invention contain an effective amount of a survivin polypeptide, a survivin peptide or a survivin peptide variant as described herein.

Vaccine compositions according to the invention may be administered to an individual in therapeutically effective amounts. The effective amount may vary according to a variety of factors such as the individual's condition, weight, sex and age. Other factors include the mode of administration.

In the following vaccine compositions are meant to encompass compositions useful for therapeutic use, including stimulating an immune response in a patient, such as a strong specific cytotoxic T cell response upon administration of said composition, an especially vaccine compositions capable of inducing a clinical response on a target lesion. It is further contemplated that the vaccine composition of the invention does not induce any systemic of local toxicity reactions or any other side effects.

To obtain vaccines or immunogenic compositions it may be required to combine the relative small survivin peptide and survivin peptide variants molecules described herein with various materials such as adjuvants, immunostimulatory components and/or carriers. Adjuvants are included in the vaccine composition to enhance the specific immune response. Thus, it is particular important to identify an adjuvant that when combined with the antigen(s) results in a vaccine composition capable of inducing a strong specific cytotoxic T cell response and most importantly a clinical response on a target lesion.

### Adjuvant

A large number of adjuvants have been described and used for the generation of antibodies in laboratory animals, such as mouse, rats and rabbits. In such setting the tolerance of side effect is rather high as the main aim is to obtain a strong antibody response.

For use and for approval for use in pharmaceuticals, and especially for use in humans it is required that the components of the vaccine composition, including the adjuvant, are well characterised. It is further required that the composition has minimal risk of any adverse reaction, such as granuloma, abscesses or fever.

In a preferred embodiment the vaccine composition is suitable for administration to a human subject, thus a preferred adjuvant are suitable for administration to a human subject.

The choice of adjuvant may further be selected by its ability to stimulate the type of immune response desired, B-cell or/and T-cell activation and the vaccine composition may be formulated to optimise distribution and presentation to the relevant lymphatic tissues.

Recently, methods including loading of appropriate antigen presenting cells with antigenic peptides have been proposed as a highly efficient way of mounting an immune response directed against cancer diseases. This method involves isolating APCs (PBLs) or APC precursor cells from the patient and loading these with the antigenic peptide, alternatively dendritic cells may be used and differentiated in vitro into APCs and loaded with the antigenic peptide prior to injection into the patient. This method is very complicated and time consuming. The use of cell cultures makes it a very inflexible vaccine composition, which requires special preparation and storage facilities. The present invention aimed at identifying a vaccine composition that is easily prepared and/or stored.

Adjuvants useful in therapeutic vaccines may be mineral salts, such as aluminium hydroxide and aluminium or calcium phosphates gels, oil emulsions and surfactant based formulations such as MF59 (microfluidised detergent stabilised oil in water emulsion), QS21 (purified saponin), AS02 (SBAS2, oil-in-water emulsion + monophosphoryl lipid A (MPL) + QS21), Montanide ISA 51 and ISA-720 (stabilised water in oil emulsion), Adjuvant 65 (containing peanut oil, mannide monooleate and aluminum monostearate), RIBI ImmunoChem Research Inc., Hamilton, Utah), particulate adjuvants, such as virosomes (unilamellar liposomal cehicles incorporating influenza haemagglutinin), AS04 (Al salt with MPL), ISCOMS (structured complex of saponins and lipids (such as cholesterol), polyactide co-glycolide (PLG), microbial derivatives (natural and synthetic) such as monophosphoryl lipid A (MPL), Detox (MPL + *M. Phlei* cell wall skeleton), AGP (RC-529 (synthetic acylated monosaccharide)), DC_chol (lipoidal immunostimulators able to self organise into liposomes), OM-174 (lipid A derivative), CpG motifs (synthetic oligonucleotides containing immunostimulatory CpG motifs), modified bacterial toxins, LT and CT, with non-toxic adjuvant effects, Endogenous human immunomodulators, e.g., hGM-CSF or hIL-12 or Immudaptin (C3d tandem array), inert vehicles such as gold particles.

QS-21 and Montanide ISA-51 adjuvants can be provided in sterile, single-use vials.

In one embodiment the vaccine composition comprises an adjuvant and survivin peptides or survivin peptides variant as described here below. In a preferred embodiment the adjuvant may be a Montanide Incomplete Seppic Adjuvant (ISA) (Seppic, Belgium) which includes Montanide ISA-51, Montanide ISA 50, Montanide ISA 70, Montanide ISA 206, Montanide ISA 708, Montanide ISA-720, Montanide ISA 763A, Montanide ISA 207, Montanide ISA 264, Montanide ISA 27, Montanide ISA 35, Montanide ISA 740, Montanide ISA 773, Montanide ISA 266, Montanide ISA 267, Montanide ISA 28, Montanide ISA 51 F, Montanide ISA 016D and Montanide IMS. The last three mentioned are still not approve for use in humans, but are potentially useful for the vaccine composition according to the invention.

Montanide ISA-51 and Montanide ISA-720 are especially for use in humans and are thus preferred and are oil-based adjuvant which must be administered as emulsions (se below).

In some embodiments, the vaccine composition may further comprise one or more additional immunostimulatory components. These include, without limitation, muramyldipeptide (MDP); e.g. N-acetyl-muramyl-L-alanyl-D-isoglutamine (ala-MDP), N-acetyl-muramyl-L-threonyl-D-isoglutamine (thr-MDP), N-acetyl-nor-muramyl-L-alanyl-D-isoglutamine (CGP 11637, nor-MDP) and N-acetyl-muramyl-L-alanyl-D-isoglutaminyl-L-alanine-2-(1'-2'-dipalmitoyl-sn-glycero-3-hydroxyphosphoryloxy)-ethylamine (CGP 19835A, MTP-PE), dimethylglycine, tuftsin, and trehalose dimycolate. monophosphoryl-lipid A (MPL), and formyl-methionine containing tri-peptides such as N-formyl-Met-Leu-Phe. Such compounds are commercially available from Sigma Chemical Co. (St. Louis, MO) and RIBI ImmunoChem Research, Inc. (Hamilton, MT), for example.

A carrier may be present independently of an adjuvant. The function of a carrier can for example be to increase the molecular weight of in particular survivin fragments in order to increase their activity or immunogenicity, to confer stability, to increase the biological activity, or to increase serum half-life. The carrier may be any suitable carrier known to the person skilled in the art. A carrier protein could be but is not limited to keyhole limpet hemocyanin, serum proteins such as transferrin, bovine serum albumin, human serum albumin, thyroglobulin or ovalbumin, immunoglobulins, or hormones, such as insulin or palmitic acid. For immunization of humans, the carrier must be a physiologically acceptable carrier acceptable to humans and safe. However, tetanus toxoid and/or diptheria toxoid are suitable carriers in one embodiment of the invention. Alternatively, the carrier may be dextrans for example sepharose.

Oil emulsions and surfactant based vaccines may be grouped as water in oil, oil in water and water in oil in water formulations.

The adjuvant used for oil in water formulation may be a mineral oil or/and a non-mineral oil and a surfactant/emulsifier. The adjuvant is mixed with the aqueous antigen composition providing the vaccine formulation.

According to the invention the oil may be metabolisable or non metabolisable or a mixture of metabolisable and non-metabolisable oils. The non mineral oils are quickly metabolised and removed from the injection site, thus they have very few side-effect but flowingly the immune response is equally small, whereas mineral oils are only partly metabolized and have a higher risk of inducing undesirable effects along with a good immune response.

It is preferred that the oil is premixed with an emulsifying agent, such as mannide mono-oleate, before the addition of the aqueous phase of the vaccine, and emulsified by use of a colloid mill or continuous mechanical or flow ultrasonic emulsifier.

More complex double emulsions (water/oil/water) may be produced by emulsifying once more in an aqueous phase containing a small amount of Tween 80.

Significant advances have been made over recent years that have seen the introduction of alternative 'ready-to-use' oil adjuvants. Oils containing esters of octadecenoic acid and anhydromannitol, for example, readily form double or mixed emulsions (Water/Oil/Water), that are both stable and of low viscosity, without the requirement of sophisticated emulsification equipment.

In an embodiment the preferred adjuvant is for a water in oil vaccine formulation.

In an embodiment the preferred oil component may be non mineral oil. In a preferred embodiment the adjuvant is selected from the group of non mineral oil based Montanide ISA (incomplete seppic adjuvants) such as Montanide ISA 708, Montanide ISA-720, Montanide ISA 763A, Montanide ISA 207, Montanide ISA 264, Montanide ISA 27 and Montanide ISA 35.

Alternatively the adjuvant may comprise non mineral oil or/and mineral oil. Thus in a preferred embodiment the adjuvant is selected from the group of non mineral oil or/and mineral oil based Montanide ISA (incomplete seppic adjuvants) such as Montanide ISA 740, Montanide ISA 773, Montanide ISA 266, Montanide ISA 267, Montanide ISA 28, and Montanide IMS whereof the latest is still not approve for use in humans.

In a most preferred embodiment the oil component is a mineral oil. In a preferred embodiment the adjuvant is selected from the group of mineral oil based Montanide ISA (incomplete seppic adjuvants (Seppic, Belgium)) such as Montanide ISA 50, Montanide ISA-51, Montanide ISA 70, Montanide ISA 206, and Montanide ISA 51 F and Montanide ISA 016D whereof the last two are still not approved for use in humans.

The composition of the mineral oil, e.g. the length of the carbon chains affects the efficiency of the adjuvant, short chains induce a strong immune response but confers local side-effects whereas the response using long chains is less but without any notable side-effects, thus the mineral oil should be well characterised and have a balance composition of long and short carbon chains. It is preferred that the mineral oil have less than 8 %, or such as less than 7 %, or such as less than 6 % and most preferably less than 5 % hydrocarbons with a chain length of less than C14.

It is preferred that the mineral oil contains no unsaturated or aromatic hydrocarbons. It is preferred that the oil component has an A.P.I. gravity of 32-40, or such as 35-37 and especially such as 36.2-36.8.

Further, it is preferred that the mineral oil has a specific gravity at 25 °C of 0.82-0.84 or such as 0.83-0.84. It is more preferred that the mineral oil has a specific gravity at 25 °C of 0.834-0.838.

A mineral oil with a viscosity at 37.8 °C (100 F) of 55-65 SSU is preferred, more preferably a mineral oil with a viscosity of 27-61 is used and it is particularly preferred that the mineral oil have a viscosity at 37.8 °C (100 F) of 59-61 SSU.

It is preferred that the mineral oil have a refractive index at 25 °C of 1.2-1.6, or such as 1.3-1.6 or such as 1.4-1.5 and most preferably 1.458-.463,

It is further preferred that the mineral oil have one or more of the following characteristics;
a) a better than minimum acid test,
b) negative for fluorescence at 360 nm,
c) negative for visible suspended matter,
d) has a minimum ASTM flash point of 295 F,
e) complies with all RN requirements for light mineral oil and ultraviolet absorption.

The mineral oil should be of pharmaceutical grade.

Drakeol 6 VR (Penreco, Texas) is a pharmaceutical grade mineral oil. Drakeol 6 VR contains no unsaturated or aromatic hydrocarbons, and has an A. P.I. gravity of 36.2-36.8, a specific gravity at 25 °C of 0.834-0.838, a viscosity at 37.8 °C (100 F) of 59-61 SSU or 10.0-10.6 centistokes, a refractive index at 25 °C of 1.458-.463, a better than minimum acid test, is negative for fluorescence at 360 nm, is negative for visible suspended matter, has an ASTM pour test value of 0-15 F, has a minimum ASTM flash point of 295 F, and complies with all RN requirements for light mineral oil and ultraviolet absorption. Less than 5 % of the mineral oil is constituted by hydrocarbons with short chains.

In a most preferred embodiment the mineral oil is Drakeol 6 VR.

### Surfactant

The surface tension of the adjuvant may be regulated by a surfactant, which thereby affects the viscosity of the adjuvant and the vaccine formulation. The toxic effect of a surfactant is correlated with the residual level of fatty acids, thus a high quality surfactant with a low level of fatty acid Is required. The surfactant should be of pharmaceutical grade.

An adjuvant according to the invention may comprise a surfactant.

In a embodiment the adjuvant Is formulated for a water in oil emulsion comprising a mineral oil and a surfactant, wherein the adjuvant comprises up to 14,5 % Vol. of said surfactant

An aspect of the specification relates to a vaccine composition comprising:
i. one or more survivin peptides or survivin peptide variants, wherein the sequence of the peptide variant, over the entire length, is at least 85 % identical to a consecutive amino acid sequence of SEQ ID NO: 23, and
ii. an adjuvant formulated for a water In oil emulsion comprising a mineral oil and a surfactant, wherein the adjuvant comprises up to 14,5 % Vol. of said surfactant,
for use as a medicament.

In a further embodiment the adjuvant comprises from 2 to 14 % Vol. of said surfactant, such as from 5 to 14 % Vol. of said surfactant, such as from 6 to 13 % Vol. of said surfactant, such as from 7 to 12 % Vol. of said surfactant or such as from 8 to 12 % Vol. of said surfactant.

It is preferred that the predominant fatty acid species of the surfactant is C18' constituting 65 to 88 % of the composition.

It is further preferred that the surfactant is an oil, such as a lipid liquid with a maximum acid value of 1.

It is furthermore preferred that the surfactant has a saponification value of 150-190, or such as 160-170, or such as 162-175, and most preferred a saponification value of 164-172.

It is furthermore preferred that the surfactant has a hydroxyl value of 70-120, or such as 80-110, or such as 80-110, 85-105, and most preferred a hydroxyl value of 89-100.

It is furthermore preferred that the surfactant has an iodine value of 40-100 or such as 50-90, or such as 60-80, or such as 65-78, and most preferred an iodine value of 67-75.

It is furthermore preferred that the surfactant has a heavy metal value of less than 40 ppm or such as 30 ppm, or such as 25 ppm, or such as 22 ppm, and most preferred a heavy metal value less than 20 ppm.

It is furthermore preferred that the surfactant has a maximum water content of 50 %, or such as 45 %, or such as 40 %, or such as 37 %, and most preferred a maximum water content of 0.35%.

It is furthermore preferred that the surfactant has a viscosity at 25 °C of such as 200-400 mPaS, or such as 225-375 mPaS, or such as 250-350 mPaS, or such as 275-325 mPaS, and most preferred a viscosity at 25 °C of about 300 mPaS.

In a preferred embodiment the surfactant is mannide oleate also known as anhydro mannitol octadecenoate.

Montanide 80 is based on oleic acid (distribution of various fatty acids) where the predominant fatty acid specie; is C18', constituting from 65 to 88 % of the composition. The oil is a lipid liquid with a maximum acid value of 1, a saponification value of 164-172, a hydroxyl value of 89-100, an iodine value of 67-75, a maximum peroxide value of 2, a heavy metal value less than 20 ppm, a maximum water content of 0.35%, a maximum colour value of 9 and a viscosity at 25 °C of about 300 mPaS.

In a most preferred embodiment the adjuvant comprises the surfactant mannide oleate, Montanide 80 (Seppic, Belgium).

In a further preferred embodiment the adjuvant comprises the mineral oil Drakeol 6VR and the surfactant mannide oleate (Montanide 80).

In a preferred embodiment the adjuvant comprises the mineral oil Drakeol 6VR and the surfactant mannide oleate, wherein the adjuvant comprises up to 14.5 % of the surfactant anhydro mannitol octadecenoate (Montanide 80).

In a more preferred embodiment the adjuvant comprises from 2 to 14 % Vol. of said surfactant anhydro mannitol octadecenoate, such as from 5 to 14 % Vol. of said surfactant anhydro mannitol octadecenoate, such as from 6 to 13 % Vol. of said surfactant anhydro mannitol octadecenoate, such as from 7 to 12 % Vol. of said surfactant anhydro mannitol octadecenoate or such as from 8 to 12 % Vol. of said surfactant anhydro mannitol octadecenoate.

In an embodiment the adjuvants is a clear yellow liquid with a density at 20 °C of about 0.7-1.0 or 0.8-0.9 or preferably about 0.85.

In an embodiment the adjuvant has a viscosity at 20 °C of less than 500 mPaS, such as less than 250mPaS or such as 25-150 mPaS or preferably about 150 mPaS.

In an embodiment the adjuvant has a maximum acid value of 0.5,

In an embodiment the adjuvant has a saponification value of 10-40, such as 12-30, such as 14-25 or preferrablyl 6-20.

In an embodiment the adjuvant has a hydroxyl value of 5-20, or such as 6-18 or such as 7-15 or preferably 9-13.

In an embodiment the adjuvant has a maximum peroxide value of 5 or 4 or 3 or preferably 2.

In an embodiment the adjuvant has an Iodine value of 1-20, such as 2-15, such as 3-12 or preferably such as 5-9.

In an embodiment the adjuvant has a maximum water content of 2 percent, such as 1.5 percent, such as 1 percent or such as preferably 0.5 percent

In an embodiment the adjuvant has a refractive Index at 25 ° between1.450 and 1.470 or between 1.455 and 1.465 or preferably between 1.461-1.463.

In an embodiment the conductivity of a 50: 50 mixture of adjuvant and saline is less than 20 µScm⁻¹, 15 µScm⁻¹, 12 µScm⁻¹ or preferably less than 10 µScm⁻¹.

Montanide ISA 51contains mannide oleate (Montanide 80) in a mineral oil solution (Drakeol 6 VR). (Montanide ISA 51 contains about 8 to 12 percent anhydro mannitol octadecenoate and about 88 to 92 percent mineral oil. Montanide ISA 51 is a clear yellow liquid with a density at 20 °C of about 0.85 and a viscosity at 20 °C of about 50 mPaS. Montanide ISA 51 is characterised by having a maximum acid value of 0.5, a saponification value of 16-20, a hydroxyl value of 9-13, a maximum peroxide value of 2, an iodine value of 5-9, a maximum water content of 0.5 percent, a refractive index at 25 °C between 1.455 and 1.465 or preferably between 1.461-1.463. The conductivity of a 50: 50 mixture of Montanide ISA 51 and saline is less than 10 µScm⁻¹.

In a more preferred embodiment the adjuvant is a Montanide incomplete seppic adjuvant.

The specification describes a vaccine composition that comprises:
i. one or more survivin peptides or survivin peptide variants, wherein the sequence of the peptide variant, over the entire length, is at least 85 % identical to a consecutive amino acid sequence of SEQ ID NO: 23, and
ii. an adjuvant formulated for a water in oil emulsion comprising a mineral oil and a surfactant, wherein the adjuvant comprises up to 14.5 % Vol. of said surfactant, wherein the adjuvant is Montanide ISA 51.

### Survivin peptide or peptide variants

Ideal targets for immunotherapy are gene products silenced in normal tissues, overexpressed in cancer cells, and directly involved in tumor cell survival and progression. Survivin potentially fulfils these features, because it suppresses apoptosis in addition to being involved in regulation of cell division. Hence, survivin is preventing cells from physiological death and, therefore, extending cell survival.

Survivin is a 16. 5 kDa cytoplasmic protein containing a single BIR and a highly charged carboxy-terminal coiled region instead of a RING finger. The coding sequence is 429 nucleotides long (SEQ ID NO: 22) including stop codons and the encoded protein Survivin is 142 amino acid long (SEQ ID NO: 23).

The present invention relates to vaccine compositions comprising one or more survivin peptides or survivin peptide variants.

In an embodiment the one or more survivin peptides or survivin peptide variants comprise the full length survivin polypeptide (SEQ ID NO: 23) consisting of 142 amino acid residues.

Preferably, the one or more survivin peptides or survivin peptide variants comprise at least 5 amino acid residues, and more preferably it comprises at least 7 amino acid residues, at least 8 amino acid residues, at least 9 amino acid residues, at least 10 amino acid residues, at least 11 amino acid residues, at least 12 amino acid residues, at least 14 amino acid residues, at least 16 amino acid residues, at least 18 amino acid residues or at least 20 amino acid residues.

In an embodiment it is further preferred that the one or more survivin peptides or survivin peptide variants consists of at the most 142 amino acid residues or such as at the most 120 amino acid residues, or such as at the most 100 amino acid residues, and preferably at the most 80 amino acid residues or such as at the most 50 amino acid residues, and more preferably it consists of at the most 20 amino acid residues, such as at the most 18, such as at the most 16, such as at the most 14, such as at the most 12, such as at the most 11, such as at the most 10 amino acid residues.

In an embodiment the vaccine composition comprises one or more peptides or peptide variants consisting of at least 5 amino acid residues and at the most 20 consecutive amino acid residues of SEQ ID NO: 23.

In an embodiment it is further preferred that the one or more survivin peptides or survivin peptide variants consists of at the most 142 consecutive amino acid residues of SEQ ID NO 23 or such as at the most 120 consecutive amino acid residues, or such as at the most 100 consecutive amino acid residues, and preferably at the most consecutive 80 amino acid residues or such as at the most 50 consecutive amino acid residues, and more preferably it consists of at the most 20 consecutive amino acid residues, such as at the most 18 consecutive amino acids, such as at the most 16 consecutive amino acids, such as at the most 14 consecutive amino acids, such as at the most 12 consecutive amino acids, such as at the most 11 consecutive amino acids, such as at the most 10 consecutive amino acid residues of SEQ ID NO 23.

In specific embodiments, the peptide consists of a heptapeptide, an octopeptide, a nonapeptide, a decapeptide or an undecapeptide, consisting of 7, 8, 9, 10, 11 consecutive amino acid residues of SEQ ID NO: 23, respectively.

The present invention also encompasses variants and functional equivalents of the survivin peptides as disclosed herein. "Functional equivalents" as used in the present context is established by means of reference to the corresponding functionality of a predetermined fragment of the sequence in question. Functional equivalence can be established by e.g. similar binding affinities to HLA class I molecules, or similar potency demonstrated by the ELISPOT assay.

Functional equivalents or variants of a survivin-derived peptide as described herein will be understood to exhibit amino acid sequences gradually differing from the preferred, predetermined sequences, as the number and scope of insertions, deletions and substitutions including conservative substitutions. This difference may be measured as a reduction in identity between a preferred, predetermined sequence and the survivin derived variant or survivin-derived functional equivalent.

The identity between amino acid sequences may be calculated using algorithms well known in the art. Fragments sharing homology with fragments comprising or consisting of consecutive survivin-derived amino acid residues are to be considered as falling within the scope of the present invention when they are preferably, over the entire length such as at least 75% identical, such as at least 80 % identical, such as at least 85 % identical, such as at least 88 % identical, at least 90% identical, such as at least 94% identical, including 95%, 96%, 97%, 98% or 99% identical with a predetermined survivin-derived peptide.

The vaccine composition according to the invention comprise one or more survivin peptides or survivin peptide variants, wherein the sequence of the peptide variant, over the entire length, is at least 85 % identical to a consecutive amino acid sequence of SEQ ID NO: 23.

An example of a peptide variant according to the invention is the peptide LLLGEFLKL (survivin₉₆₋₁₀₄L2,) (SEQ ID NO: 4) where in the peptide variant is 8/9 *100% (89 %) identical with the survivin sequence "LTLGEFLKL".

The denomination used above to describe peptide variants is used throughout the present application: A letter followed by a number e.g. L2 indicates the variant amino acid (L) that has replaced the amino acid otherwise present in position 2 of the given peptide. The given peptide is indicated either by one number, this number indicating the number of the first amino acid of the peptide in relation to the full length survivin sequence (survivin₉₆ or Sur96 starts with residue number 96 of survivin) or two numbers, where the second number gives the end residue of the peptide in regards to survivin.

The peptide variants may be derived from the known sequence of survivin, e. g. the sequence disclosed in US 6.245. 523 (SEQ ID NO: 23 herein). The selection of peptides potentially having the ability to bind to a particular HLA molecule can be made by using the alignment of known sequences that bind to a given particular HLA molecule to reveal the predominance of a few related amino acids at particular positions in the peptides. Such predominant amino acid residues are also referred to herein as "anchor residues" or "anchor residue motifs". By following such a relatively simple procedure based on known sequence data that can be found in accessible databases, peptides can be derived from the survivin protein molecule, which are likely to bind to the particular HLA molecule. Representative examples of such analyses for a range of HLA molecules are given in the below table 2:

**Table 2. Primary anchor residue motifs employed.**

| HLA allele | Position 1 | Position 2 | Position 3 | Position 5 | Position 6 | Position 7 | C-terminal |
|---|---|---|---|---|---|---|---|
| HLA-A1 | | T,S | D,E | | | L | Y |
| HLA-A2 | | L, M | | | V | | L,V |
| HLA-A3 | | L,V,M | F,Y | | | | K, Y, F |
| HLA-A11 | | V,I,F,Y | M,L,F,Y, | | | | K, R |
| HLA-A23 | | I,Y | | | | | W,I |
| HLA-A24 | | Y | | I,V | F | | I,L,F |
| HLA-A25 | | M,A,T | I | | | | W |
| HLA-A26 | E,D | V,T,I,L,F | | | I,L,V | | Y,F |
| HLA-A28 | E,D | V,A,L | | | | | A,R |
| HLA-A29 | | E | | | | | Y,L |
| HLA-A30 | | Y,L,F,V | | | | | Y |
| HLA-A31 | | | L,M,F,Y | | | | R |
| HLA-A32 | | I,L | | | | | W |
| HLA-A33 | | Y,I,L,V | | | | | R |
| HLA-A34 | | V,L | | | | | R |
| HLA-A66 | E,D | T,V | | | | | R,K |
| HLA-A68 | E,D | T,V | | | | | R,K |
| HLA-A69 | | V,T,A | | | | | V,L |
| HLA-A74 | | T | | | | | V,L |
| HLA-B5 | | A,P | F,Y | | | | I,L |
| HLA-B7 | | P | | | | | L,F |
| HLA-B8 | | | K | K,R | | | L |
| HLA-B14 | | R,K | | | | | L,V |
| HLA-B15 (B62) | | Q,L,K,P, H,V,I,M, S,T | | | | | F,Y,W |
| HLA-B17 | | | | | | | L,V |
| HLA-B27 | | R | | | | | Y, K,F,L |
| HLA-B35 | | P | | | | | I, L, M, Y |
| HLA-B37 | | D,E | | | | | I,L,M |
| HLA-B38 | | H | D,E | | | | F,L |
| HLA-B39 | | R,H | | | | | L,F |
| HLA-B40 (B60,61) | | E | F,I,V | | | | L,V,A,W ,M,T,R |
| HLA-B42 | | L,P | | | | | Y,L |
| HLA-B44 | | E | | | | | F,Y,W |
| HLA-B46 | | M,I,L,V | | | | | Y,F |
| HLA-B48 | | Q,K | | | | | L |
| HLA-B51 | | A,P,G | | | | | F,Y,I,V |
| HLA-B52 | | Q | F,Y | | | | I,V |
| HLA-B53 | | P | | | | | W,F,L |
| HLA-B54 | | P | | | | | |
| HLA-B55 | | P | | | | | A,V |
| HLA-B56 | | P | | | | | A,V |
| HLA-B57 | | A,T,S | | | | | F,W,Y |
| HLA-B58 | | A,T,S | | | | | F,W,Y |
| HLA-B67 | | P | | | | | L |
| HLA-B73 | | R | | | | | P |
| HLA-Cw1 | | A,L | | | | | L |
| HLA-Cw2 | | A,L | | | | | F,Y |
| HLA-Cw3 | | A,L | | | | | L,M |
| HLA-Cw4 | | Y,P,F | | | | | L,M,F,Y |
| HLA-Cw6 | | | | | | | L,I,V,Y |
| HLA-Cw6 | | Y | | | | | L,Y,F |
| HLA-Cw8 | | Y | | | | | L,I, |
| HLA-Cw16 | | A,L | | | | | L,V |

Thus, as an example, nonapeptides potentially having the ability to bind to HLA-A1 would have one of the following sequences: Xaa-T-D-Xaa-Xaa-Xaa-L-Xaa-Y, Xaa-T-E-Xaa-Xaa-Xaa-L-Xaa-Y, Xaa-S-D-Xaa-Xaa-Xaa-L-Xaa-Y or Xaa-S-E-Xaa-Xaa-Xaa-L-Xaa-Y (Xaa indicating any amino acid residue). In a similar manner, sequences potentially having the ability to bind to any other HLA molecule can be designed.

It will be appreciated that the person of ordinary skill in the art will be able to identify further "anchor residue motifs" for a given HLA molecule.

Thus a peptide variant according to the invention include peptides comprising a sequence including any of the amino acid residues listed in Table 2 for each of the specific HLA.

Alternatively, the differences may be measured directly by comparing the number of substitutions in a peptide compared to a consecutive amino acid sequence of survivin. Thus in an embodiment the vaccine comprises a survivin peptide variant consisting of 7-20 consecutive amino acid comprising one or two amino acid substitutions compared to a consecutive amino acid sequence of SEQ ID NO: 23. In a more preferred embodiment the vaccine composition comprise a survivin peptide variant consisting of 7-12 consecutive amino acid comprising one amino acid substitutions compared to a consecutive amino acid sequence of SEQ ID NO: 23.

The binding groove of MHC class 1 molecules perfectly suits a peptide of 9 or 10 amino acids. In a more preferred embodiment the vaccine composition comprise one or more survivin peptides or survivin peptide variants consisting of 9 or 10 amino acid residues.

Based on the sequence of the selected survivin protein, the one or more survivin peptides or survivin peptide variants may be derived by any appropriate chemical or enzymatic treatment of the survivin starting material that results in a peptide of a suitable size as indicated above, or it can be synthesised by any conventional peptide synthesis procedures with which the person of ordinary skills in the art is familiar.

The peptide of the invention may have a sequence which is a native sequence of the survivin protein from which it is derived. However, peptides having a higher affinity to any given HLA molecule may be derived from such a native sequence by modifying the sequence by substituting, deleting or adding at least one amino acid residue, e. g. on the basis of the procedure described above whereby anchor residue motifs in respect of the given HLA molecule are identified (see table 2).

Thus, in an embodiment the vaccine composition comprises one or more survivin peptide or peptide variants, wherein the sequence of the survivin peptide variant(s) may be derived from a native sequence by substituting, deleting or adding at least one amino acid residue, whereby a peptide having anchor residue motifs for a given HLA molecule is obtained.

Accordingly, to increase the immunogenicity of survivin-derived peptides, amino acid substitutions can be introduced at anchor positions, but not at TCR contact residues, to increase peptide binding to the HLA class I molecule. This has resulted in more immunogenic epitopes, e.g. , this has enhanced the capacity to induce cancer-reactive CTL and it has been demonstrated to be more suitable for the induction of clinically meaningful CTL responses. Importantly, however, the target cancer cells do only express and present the native survivin-derived peptide on the cell-surface. In that respect, it is of crucial importance that therapy-induced CTL specific for the modified survivin-derived peptides cross-react with the native analogues.

In an embodiment the one or more survivin peptide or survivin peptide variants is/are restricted to at least one of the MHC Class I molecules selected from the group of: HLA-A1, HLA-A2, HLA-A3, HLA-A11, HLA-A23, HLA-A24, HLA-A25, HLA-A26, HLA-A28, HLA-A29, HLA-A30, HLA-A31, HLA-A32, HLA-A33, HLA-A34, HLA-A66, HLA-A68, HLA-A69, HLA-A74, HLA-B5, HLA-B7, HLA-B8, HLA-B14, HLA-B15 B62), HLA-B17, HLA-B27, HLA-B35, HLA-B37, HLA-B38, HLA-B39, HLA-B40 (B60,61), HLA-B42, HLA-B44, HLA-B46, HLA-B48, HLA-B51, HLA-B52, HLA-B53, HLA-B54, HLA-B55, HLA-B56, HLA-B57, HLA-B58, HLA-B67, HLA-B73, HLA-Cw1 HLA-Cw2, HLA-Cw3, HLA-Cw4, HLA-Cw6, HLA-Cw6.

Thus, in a even further embodiment the one or more peptide(s) is/are restricted to at least one of the MHC Class I molecules selected from the group of: HLA-A1, HLA-A2, HLA-A3, HLA-A11, HLA-A24, HLA-B7, HLA-B35, HLA-B44, HLA-B8, HLA-B15, HLA-B27 and HLA-B51, HLA-Cw1, HLA-Cw2, HLA-Cw3, HLA-Cw4, HLA-Cw5, HLA-Cw6, HLA-Cw7 and HLA-Cw16.

In a specifically preferred embodiment the one or more peptide(s) is/are restricted to at least one of the MHC Class I molecules selected from the group of: HLA-A1, HLA-A2, HLA-B7 and HLA-B35.

The vaccine according to the invention may comprise two or more peptides with the same tissue specificity as the effeciency of individual peptides may vary in different individuals. Thus in an embodiment two or more survivin peptide or survivin peptide variants are restricted to a MHC Class I HLA molecule selected from the group of: HLA-A1, HLA-A2, HLA-A3, HLA-A11, HLA-A23, HLA-A24, HLA-A25, HLA-A26, HLA-A28, HLA-A29, HLA-A30, HLA-A31, HLA-A32, HLA-A33, HLA-A34, HLA-A66, HLA-A68, HLA-A69, HLA-A74, HLA-B5, HLA-B7, HLA-B8, HLA-B14, HLA-B15 B62), HLA-B17, HLA-B27, HLA-B35, HLA-B37, HLA-B38, HLA-B39, HLA-B40 (B60,61), HLA-B42, HLA-B44, HLA-B46, HLA-B48, HLA-B51, HLA-B52, HLA-B53, HLA-B54, HLA-B55, HLA-B56, HLA-B57, HLA-B58, HLA-B67, HLA-B73, HLA-Cw1 HLA-Cw2, HLA-Cw3, HLA-Cw4, HLA-Gw8,HLA-Gw8.

In a further embodiment the one or more survivin peptide or survivin peptide variants is/are restricted to at least one of the MHC Class I HLA-A molecule selected from the group of: HLA-A1, HLA-A2, HLA-A3,HLA-A9, HLA-A 10, HLA- 11, HLR-Aw 19, HLA A23 (9),HLA-424 (9), HLA-A25 (10), HLA-A26(10), HLA-A28, HLA-A29(w19),HLA-A30 (w19), HLA-A31 (w19), HLA-A32 (wI9), HLA-Aw33(w19), HLA-Aw34(10), HLA-Aw36, HLA-Aw43, HLA-Aw66 (10), HLA-Aw68(28), HLA-A69 (28). More simple designations are also used throughout the literature, where only the primary numeric designation is used, e. g.HLA-A19 or HLA-A24 instead ofHLA-AwI9 and HLA-A24 (9), respectively. In specific embodiments, the one or more survivin peptides or survivin peptide variants is/are restricted to a MHC Class I HLA species selected from the group consisting of; HLA-A1, HLA-A2, HLA-A3, HLA-A11 and HLA-A24.

The specification describes that
one or more survivin peptides or survivin peptide variants is/are restricted to HLA-A1.

The specification describes that
one or more survivin peptides or survivin peptide variants is/are a HLA-1 restricted peptide having a sequence selected from the following group; MAEAGFIHY (SEQ ID NO: 17) (survivin₃₈₋₄₆Y9, survivin₃₈₋₄₆ with a "Y" substituting a "C" at position 9), PTENEPDLAY (SEQ ID NO: 18) (survivin₄₇₋₅₈Y10, survivin₄₇₋₅₆ with a "Y" substituting "Q" at postion10), QFEELTLGEF (SEQ ID NO: 15) (survivin₉₂₋₁₀₁) and FTELTLGEF (SEQ ID NO: 16)(survivin₉₃₋₁₀₁T2, survivin₉₃₋₁₀₁ with a "T" substituting "E" at position 2). The designations in brackets indicate the positions of the residues in the survivin protein as disclosed in US6,245,523 and the amino acid change in the peptide.

In a specific embodiment the one or more survivin peptides or survivin peptide variants is/are restricted to HLA-A2.

In further specific embodiments, the one or more survivin peptides or survivin peptide variants is/are an HLA-A2 restricted survivin-derived peptide having a sequence selected from the following: FLKLDRERA (survivin₁₀₁₋₁₀₈) (SEQ ID NO: 1), TLPPAWQPPL(survivin₅₋₁₄) (SEQ ID NO: 2), ELTLGEFLKL(survivin₉₅₋₁₀₄) (SEQ ID NO: 3), LLLGEFLKL (survivin₉₅₋₁₀₄L2, survivin₉₆₋₁₀₄ with a substitution of "T" with an "L" in position 2) (SEQ ID NO: 4) and LMLGEFLKL survivin₉₆₋₁₀₄M2 (surviving₉₆₋₁₀₄ with a substitution of "T" with a "M" in position 2 (SEQ ID NO: 5).

The specification describes that
the one or more survivin peptides or survivin peptide variants may also be a HLA-A3 restricted peptide such as RISTFKNWPK (Sur18K10) Survivin₁₈₋₂₇ with a "F" changed to a "K" at position 10, (SEQ ID NO: 20) and/or HLA-A11 restricted peptides such as DLAQCFFCFK (survivin53-62) (SEQ ID NO: 19), DVAQCFFCFK (Sur53/V2) (SEQ ID NO: 45), DFAQCFFCFK (Sur53/F2) (SEQ ID NO: 46), DIAQCFFCFK (Sur53/I2) (SEQ ID NO: 47), and/or a HLA-A2 restricted peptide such as RISTFKNWPFL (survivin18-28) (SEQ ID NO: 21), and/or HLA-A24 restricted peptides such as STFKNWPFL (Sur20-28) (SEQ ID NO: 41), or with a "T" changed to a "Y" at position 2: SYFKNWPFL (Sur20-28/Y2) (SEQ ID NO: 48).

The specification describes that
the one or more survivin peptides or survivin peptide variants is/are restricted to a MHC Class I HLA-B molecule including any of the following: HLA-B5, HLA-B7,HLA-B8,HLA-B12, HLA-B13, HLA-B14, HLA-B15, HLA-B16, HLA-B17, HLA-B118, HLA-B21, HLA-Bw22, HLA-B27, HLA-B35, HLA-B37, HLA-B38, HLA-B39,HLA-B40, HLA-Bw41, HLA-Bw42, HLA-B44, HLA-B45, HLA-Bw46 and HLA-Bw47. In specific embodiments, the MHC Class I HLA-B species to which the one or more survivin peptides or survivin peptide variants is/are capable of binding is selected from the group of: HLA-B7, HLA-B8, HLA-B15, HLA-B27, HLA-B35, HLA-B44, HLA-B51 and HLA-B58.

The specification describes that
the one or more survivin peptides or survivin peptide variants is/are restricted to HLA-B7 or HLA-B35.

The specification describes that
the one or more survivin peptides or survivin peptide variants is/are restricted to HLA-B7.

The specification describes that
the one or more survivin peptides or survivin peptide variants is/are an HLA-B7 binding survivin derived peptide having a sequence selected from the following: LPPAWQPFL (surviving) (SEQ ID NO:10), QPFLKDHRI (survivin₁₁₋₁₉) (SEQ ID NO: 11), CPTENEPDL (survivin₅₁₋₅₉) (SEQ ID NO: 6), TPERMAEAGF (survivin₃₄₋₄₃) (SEQ ID NO: 12), APPAWQPFL (survivin₆₋₁₄A1) (SEQ ID NO:13), RPPAWQPFL (survin₆₋₁₄R1) (SEQ ID NO:14). RAIEQLAAM (Sur133-141) (SEQ ID NO: 44), TAKKVRRAI (Sur127-135) (SEQ ID NO: 49), RPIEQLAAM (Sur133P2) (SEQ ID NO: 50) or TPKKVRRAI (Sur127P2) (SEQ ID NO: 51). APPAWQPFL (SEQ ID NO: 13) is a sequence derived from survivin₈₋₁₄ by substituting "L" in position 1 of the peptide with an "A" and RPPAWQPFL (SEQ ID NO: 14) is derived from surviving₈₋₁₄ by substituting an "L" in position 1 of the peptide with a "R".

In a specific embodiment the specification describes that the one or more survivin peptides or survivin peptide variants is/are restricted to HLA-B35.

In further specific embodiments the specification describes that, the one or more survivin peptides or survivin peptide variants is/are an HLA-B35-restricted survivin-derived peptide having a sequence selected from the following: CPTENEPDL (survivin₄₈₋₆₄) (SEQ ID NO: 6), EPDLAQCFF(survivin₅₁₋₅₉) (SEQ ID NO: 7), CPTENEPDY (survivin₄₆₋₅₄Y9) (SEQ ID N0: 8) and EPDLAQCFY (survivin₅₁₋₅₉Y9) (SEQ ID NO: 9). The designations in brackets indicate the positions of the residues In the survivin protein as disclosed in US6,245,523. CPTENEPDY (SEQ ID NO: 8) is a sequence derived from survivin₄₆₋₅₄ by substituting "L" in the C-terminal of the peptide with a "Y" and EPDLAQCFY (SEQ ID NO: 9) is derived from survivin₅₁₋₅₉ by substituting an "F" residue in the C-terminal with a "Y".

In a specific embodiment described in the specification the one or more survivin peptides or survivin peptide variants is/are restricted to HLA-B51. Specifically, the survivin peptide is an HLA-B51-restricted survivin-derived peptide having the sequence: RAIEQLAAM (Sur133-141) (SEQ ID NO: 44). This peptide is also HLA-B7 restricted.

In a specific embodiment described in the specification the one or more survivin peptides or survivin peptide variants is/are restricted to HLA-B27.

In further specific embodiments described in the specification, the one or more survivin peptides or survivin peptide variants is/are an HLA-B27-restricted survivin-derived peptide having a sequence selected from the following: ERMAEAGFI (Sur36-44) (SEQ ID NO: 43), ERAKNKIAK (Sur107-115) (SEQ ID NO: 52), DRERAKNKI (Sur105-113) (SEQ ID NO: 53), KEFEETAKK (Sur122-130) (SEQ ID NO: 54), ERMAEAGFL (Sur36/L9) (SEQ ID NO: 55), ERMAEAGFF (Sur36/F9) (SEQ ID NO: 56), ERMAEAGFR (Sur36/R9) (SEQ ID NO: 57), ERMAEAGFK (Sur36/K9) (SEQ ID NO: 58) or KRFEETAKK (Sur122/R2) (SEQ ID NO: 59).

In a specific embodiment described in the specification the one or more survivin peptides or survivin peptide variants is/are restricted to HLA-B44.

In further specific embodimentsdescribed in the specification, the one or more survivin peptides or survivin peptide variants is/are an HLA-B44-restricted survivin-derived peptide having a sequence selected from the following: KETNNKKKEY (Sur115Y10) (SEQ ID NO: 42), KETNNKKKEF (Sur115-124) (SEQ ID NO: 60), EELTLGEFL (Sur94-102) (SEQ ID NO: 61) or EELTLGEFY (Sur94Y9) (SEQ ID NO: 62).

In a specific embodiment described in the specification the one or more survivin peptides or survivin peptide variants is/are restricted to HLA-B8.

In further specific embodiments described in the specification, the one or more survivin, peptides or survivin peptide variants is/are an HLA-B8-restrided survivin-derived peptide having a sequence selected from the following: ISTFKNWPFL (Sur19-28) (SEQ ID NO: 63), ISKFKNWPFL (Sur19/K3) (SEQ ID NO: 64), LSVKKQFEEL (Sur87-96) (SEQ ID NO: 65), LSKKKQFEEL (Sur87/K3) (SEQ ID NO: 66), RAKNKIAKET (Sur108-117) (SEQ ID NO: 67), RAKNKIAKEL (Sur108/L10) (SEQ ID NO: 68), NNKKKEFEET (Sur118) (SEQ ID NO: 69), NNKKKEFEEL (Sur118/L10) (SEQ ID NO: 70), QPKLKDHRI (Sur11/K3) (SEQ ID NO: 71), FLKDHRIST (Sur13) (SEQ ID NO: 72), FLKDKRIST (Sur13/K5) (SEQ ID NO: 73), FLKDHRISL (Sur13/L9) (SEQ ID NO: 74), AFLSVKKQF (Sur85) (SEQ ID NO: 75), AFLSVKKQF (Sur85/K3) (SEQ ID NO: 76), AFLSKKKQF (Sur85/K5) (SEQ ID NO: 77), AFLSVKKQL (Sur85/L9) (SEQ ID NO: 78), FLSVKKQFE (Sur86) (SEQ ID NO: 79), FLSVKKQFL (Sur86/L9) (SEQ ID NO: 80), FLKVKKQFE (Sur86/K3) (SEQ ID NO: 81), SVKKQFEEL (Sur88) (SEQ ID NO: 82), SVKKKFEEL (Sur88/K5) (SEQ ID NO: 83), FLKLKRERA (Sur101/K5) (SEQ ID NO: 84), FLKLDRERL (Sur101/L9) (SEQ ID NO: 85), TAKKKRRAI (Sur127/K5) (SEQ ID NO: 86) or TAKKVRRAL (Sur127/L9) (SEQ ID NO: 87), or TAKKVRRAI (Sur127) (SEQ ID NO: 49, FLKLDRERA (Sur101) (SEQ ID NO: 1) or QPFLKDHRI (Sur11) (SEQ ID NO: 11).

In a specific embodiment described in the specification the one or more survivin peptides or survivin peptide variants is/are restricted to HLA-B15.

In further specific embodiments described in the specification, the one or more survivin peptides or survivin peptide variants is/are an HLA-B15-restricbed survivin-derived peptide having a sequence selected from the following: TLPPAWQPF (Sur5) (SEQ ID NO: 88), TLPPAWQPY (Sur5/Y9) (SEQ ID NO: 89), WQPFLKDHRI (Sur10) (SEQ ID NO: 90), WQPFLKDHRY (Sur10/Y9) (SEQ ID NO: 91), FLKDHRISTF (Sur13) (SEQ ID NO: 92), FLKDHRISTY (Sur13/Y9) (SEQ ID NO: 93), RISTFKNWPF (Sur18) (SEQ ID NO: 94), RLSTFKNWPF (Sur18/L2) (SEQ ID NO: 95), DLAQCFFCF (Sur53) (SEQ ID NO: 96), DLAQCFFCY(Sur53/Y9) (SEQ ID NO: 97), ISTFKNWPF (Sur19) (SEQ ID NO: 98), IQTFKNWPF (Sur19/Q2) (SEQ ID NO: 99), ILTFKNWPF (Sur19/L2) (SEQ ID NO: 100), RMAEAGFIY (Sur3T/Y9) (SEQ ID NO: 101), RMAEAGFIF (Sur37/F9) (SEQ ID NO: 102), RLAEAGFIY (Sur37/L2Y9) (SEQ ID NO: 103), KKHSSGCAF (Sur78) (SEQ ID NO: 104), KQHSSGCAF (Sur78/Q2) (SEQ ID NO: 105), KLHSSGCAF (Sur78/L2) (SEQ ID NO: 106), RAIEQLAAY (Sur133/Y9) (SEQ ID NO: 107) or RLIEQLAAM (Sur133/L2) (SEQ ID NO: 108) or RAIEQLAAM (Sur133) (SEQ ID NO: 44).

In a specific embodiment described in the specification the one or more survivin peptides or survivin peptide variants is/are restricted to HLA-B58.

In further specific embodiments described in the specification, the one or more survivin peptides or survivin peptide variants is/are an HLA-B58-restricted survivin-derived peptide having a sequence selected from the following: PTLPPAWQPF Sur5 (SEQ ID NO: 109), CTPERMAEAGF (Sur33) (SEQ ID NO: 110), ETNNKKKEF (Sur116) (SEQ ID NO: 111), ISTFKNWPF (Sur19) (SEQ ID NO: 98), GAPTLPPAW (Sur2) (SEQ ID NO:112) or CAFLSVKKQF (Sur84) (SEQ ID NO: 113).

In further useful embodiments described in the specification, the one or more survivin peptides or survivin peptide variants is/are a peptide, which is restricted to a MHC Class I HLA-C molecule selected from the group of: HLA-Cw1, HLA-Cw2, HLA-Cw3, HLA-Cw4, HLA-Cw5, HLA-Cw6, HLA-Cw7 and HLA-Cw16.

Thus in an embodiment the one or more survivin peptides or survivin peptide variants is/are a peptide comprises one or more post-translational modifications.

The peptides may comprise any type of modifications. Nearly 200 structurally distinct covalent modifications have been identified thus far, ranging in size and complexity from conversion of amides to carboxylic acids, to the attachment of multiple complex oligosaccharides. Such modifications include phosphorylation, acetylation, ubiquination, lipidation (acetylation, prenylation, farnesylation, geranylation, palmitoylation, myristoylation), methylation, carboxylation, sulfunation and O- or N-glycosylations.

It has been shown that exposure of breast carcinoma MCF-7 or cervical carcinoma HeLa cells to anticancer agents including Adriamycin, Taxol, or UVB resulted in a 4-5-fold increased survivin expression. Changes in survivin levels after anticancer treatment did not involve modulation of survivin mRNA expression and were independent of de novo gene transcription. Conversely, inhibition of survivin phosphorylation on Thr34 by the cyclin-dependent kinase inhibitor flavopiridol resulted in loss of survivin expression, and nonphosphorylatable survivinThr₃₄ to Ala mutant exhibited accelerated clearance as compared with wild-type survivin. Sequential ablation of survivin phosphorylation on Thr34 enhanced tumor cell apoptosis induced by anticancer agents independently of p53 and suppressed tumor growth without toxicity in a breast cancer xenograft model in vivo. These data suggest that Thr34 phosphorylation critically regulates survivin levels in tumor cells and that sequential ablation of p34 kinase activity may remove the survivin viability checkpoint and enhance apoptosis in tumor cells.

Accordingly, it is contemplated that the survivin and survivin-derived peptides of the invention encompass phosphorylated peptides. Native survivin phosphopeptide antigens may be identified by scanning for the presence of MHC peptide binding motifs around the phosphorylation site Thr34. Thus, possible survivin-derived phosphopeptide sequences include TPERMAEAGF (SEQ ID NO: 114), a putative HLA-B35-and/or HLA-B7-and/or a HLA-B51-restricted peptide antigen. Additional native phosphopeptides encompassed herein include: HLA-A2: CACTPERMA (SEQ ID NO:115), and CTPERMAEA (SEQ ID NO: 116),; HLA-A3: FLEGCACTP (SEQ ID NO: HLA-B7-and/or a HLA-B51-restricted peptide antigen. Additional native phosphopeptides encompassed herein include: HLA-A2: CACTPERMA (SEQ ID NO:115), and CTPERMAEA (SEQ ID NO: 116),; HLA-A3: FLEGCACTP (SEQ ID NO: 117),; HLA-B7/HLA-B35/HLA-B51: WPFLEGCACT (SEQ ID NO: 118), (Phoshorylated Thr residue marked in bold).

It Is well known, that the different HLA molecules are of different prevalence in the major human populations. Accordingly, there is a requirement for identifying peptide epitopes restricted to several HLA class I molecules to extend the patient cohort that can be treated according to the methods of the present invention. The characterisation of multiple survivin epitopes with different HLA restriction elements broadens the clinical potential of this target antigen in two important ways: (i) It increases the number of patients eligible for immunotherapy based on survivin-derived peptides. The HLA-A2 antigen is expressed by around 50% of the Caucasian and Asian populations, the HLA-A1 and HLA-A3 antigens are both expressed by around 25% of Caucasians and 5% of Asians, whereas the HLA-A11 antigen is expressed by around 15% of Caucasians and 30 % of Asians. Even though these numbers cannot be summed up due to co-expression, a combination of peptides restricted by a multiplicity of these would certainly encompass most cancer patients, (ii) The collective targeting of several restriction elements in each patient is likely to decrease the risk of immune escape by HLA-allele loss. Loss of a single HLA allele is a significant component of MHC alterations described for cancer cells, whereas total loss of Class I expression is a rather infrequent event. Thus, with the identification of survivin epitopes restricted to different HLA alleles, it Is now possible to target more than one HLA-molecule simultaneously in patients with allelic overlap.

Although many potential peptides for use in vaccine compositions can be predicted the, actual identification of peptides and vaccine composition capable of obtaining useful response requires testing of multiple parameters. The invention describes a vaccine composition that is particular effective. The vaccine composition according to the invention includes survivin peptides and survivin peptides variants.

Example of a presently preferred multiepitope vaccines described in the specification include "tailor made" combinations of survivin-derived peptide epitopes depending of the tissue type of the given patient, e.g., a subject carrying HLA-1, HLA-A2, HLA-A3, and HLA-B35 phenotypes could be vaccinated with a vaccine comprising the following peptides, ELTLGEFLKL(survivin₉₅₋₁₀₄) (SEQ ID NO: 3), LMLGEFLKL survivin₉₈₋₁₀₄M2 ((SEQ ID NO: 5), CPTENEPDY (survivin₄₈₋₅₄Y9) (SEQ ID N0: 8) and EPDLAQCFY (surviving₅₁₋₅₉Y9) (SEQ ID NO: 9) and/or RISTFKNWPK (Sur18K10) (SEQ ID NO: 20)

Alternatively, the epitope may be selected based on the prevalence of the various HLA phenotypes in a given population. As an example, HLA-A2 is the most prevalent phenotype in the Caucasian population, and therefore, a peptide binding to HLA-A2 will be active in a large proportion of that population.

However, the vaccine composition according to the invention may also contain a combination of two or more survivin peptides or survivin peptide variants, each interacting specifically with a different HLA molecule so as to cover a larger proportion of the target population. Thus, as examples, the vaccine composition may contain a combination of a peptide restricted to a HLA-A molecule and a peptide restricted to a HLA-B molecule, e. g. including those HLA-A and HLA-B molecules that correspond to the prevalence of HLA phenotypes In the target population, such as e.g. HLA-A2 and HLA-B35. Additionally, the vaccine composition may comprise a peptide restricted to an HLA-C molecule. Other combinations according to the invention include peptide restricted to HLA-A1 and HLA-A2, or peptide restricted to HLA-A1 and HLA-B35. Additionally three peptide with different specificity may be used, such a combination of peptides restricted to HLA-A1, HLA-A2 and HLA-B35,

It may be advantageous to include one HLA-B7 restricted peptide or two peptides such as a HLA-A1 and a HLA A2 binding peptides or such as a HLA-A1 and a HLA-B35 binding peptide in the vaccine composition.

An aspect of the specification relates to a HLA-B7 restricted peptide such as LPPAWQPFL (survivin₆₋₁₄) (SEQ ID NO: 10), QPFLKDHRI (survivin₁₁₋₁₉) (SEQ ID NO: 11), CPTENEPDL (survivin₅₁₋₅₉) (SEQ ID NO: 6), TPERMAEAGF (survivin₃₄₋₄₃) (SEQ ID NO: 12), APPAWQPFL (survivin₈₋₁₄A1) (SEQ ID NO: 13) or RPPAWQPFL (survivin₆₋₁₄R1) (SEQ ID NO: 14).

In one embodiment described in the specification the HLA-B7 restricted peptide is APPAWQPFL (survivin₆-₁₄A1) (SEQ ID NO: 13) or RPPAWQPFL (survivin₆₋₁₄R1) (SEQ ID NO: 14). In a specific embodiment the HLA-B7 restricted peptide is APPAWQPFL (survivin₆₋₁₄A1) (SEQ ID NO: 13) In a different specific embodiment the HLA-B7 restricted peptide is RPPAWQPFL (survivin₆₋₁₄R1) (SEQ ID NO: 14).

An aspect of the specification relates to a vaccine composition comprising one or more survivin peptide or peptide variants, wherein the sequence of the peptide variant, over the entire length, is at least 85 % identical to a consecutive amino acid sequence of SEQ ID NO: 23, and wherein the composition comprises
i. a HLA-B7 binding peptide and/or a HLA-A1 and a HLA-A2 restricted peptide and/or a HLA-A1 and a HLA-B35 restricted peptide
ii. and any of the adjuvants mentioned above, such as Montanide ISA 51.

In specific preferred embodiments described in the specification the vaccine composition comprises:
i. a peptide comprising APPAWQPFL (SEQ ID NO: 13) and consisting of at the most 15, preferably 10 amino acids,
   and/or
   a peptide comprising RPPAWQPFL (SEQ ID NO: 14 and consisting of at the most 15, preferably 10 amino acids,
   and/or
   a peptide comprising FTELTLGEF (SEQ ID NO: 16) and consisting of at the most 15, preferably 10 amino acids and a peptide comprising LMLGEFLKL (SEQ ID NO: 5) and consisting of at the most 15, preferably 10 amino acids,
   and/or,
   a peptide comprising FTELTLGEF (SEQ ID NO: 16) and consisting of at the most 15, preferably 10 amino acids and a peptide comprising EPDLAQCFY (SEQ ID NO: 9) and consisting of at the most 15, preferably 10 amino acids,
   and/or
   a peptide comprising LMLGEFLKL (SEQ ID NO: 5) and consisting of at the most 15, preferably 10 amino acids and a peptide comprising EPDLAQCFY (SEQ ID NO: 9) and consisting of at the most 15, preferably 10 amino acids,
ii. any of the adjuvants mentioned above, such as Montanide ISA 51.

It may further improve the efficacy of the vaccine composition to include three or more survivin peptide or survivin peptide variants and in particular if the peptides are restricted to different HLA molecules.

In an embodiment described in the specification the vaccine composition comprises,
a) three or more survivin peptide or survivin peptide variants, wherein the sequence of the peptide variant, over the entire length, is at least 85 % identical to a consecutive amino acid sequence of SEQ ID NO: 23,
   i. and wherein at least one peptide or peptide variant is selected from the group of HLA-A1 binding peptides,
   ii. and wherein at least one peptide or peptide variant is selected from the group of HLA-A2 binding peptides,
   iii. and wherein at least one peptide or peptide variant is selected from the group of HLA-B35 binding peptides
b) and any of the adjuvants) mentioned above, such as Montanide ISA 51.

In an embodiment described in the specification the HLA-A1 binding peptide, the HLA-A2 binding peptide and/or the HLA-B35 binding peptide preferably consist of at the most 15, such as at the most 14, 13, 12, 11, and most preferably at the most 10 amino acids.

In a particular embodiment described in the specification the vaccine composition comprises the HLA-A1 restricted peptide FTELTLGEF (SEQ ID NO: 16). In a second specific embodiment the vaccine composition comprises the HLA-A2 binding peptide LMLGEFLKL (SEQ ID NO: 5 and in a third specific embodiment the vaccine composition comprises the HLA-B35 binding peptide EPDLAQCFY (SEQ ID NO: 9).

In a most particular embodiment described in the specification the vaccine composition comprises the HLA-A1 restricted peptide FTELTLGEF (SEQ ID NO: 16), the HLA-A2 binding peptide LMLGEFLKL (SEQ ID NO: 5) and the HLA-B35 binding peptide EPDLAQCFY (SEQ ID NO: 9).

In an embodiment described in the specification the HLA-A1 binding peptide, the HLA-A2 binding peptide and/or the HLA-B35 binding peptide as mentioned here above preferably consist of at the most 15, such as at the most 14, 13, 12, 11, and most preferably at the most 10 amino acids.

It may further improve the efficacy of the vaccine composition to include seven or more survivin peptide or survivin peptide variants and in particular if the peptides are restricted to different HLA molecules.

In an embodiment described in the specification the vaccine composition comprises,
a) seven or more survivin peptide or survivin peptide variants, wherein the sequence of the peptide variant, over the entire length, is at least 85 % identical to a consecutive amino acid sequence of SEQ ID NO: 23,
   viii. and wherein at least one peptide or peptide variant is selected from the group of HLA-A1 binding peptides,
   ix. and wherein at least one peptide or peptide variant Is selected from the group of HLA-A2 binding peptide,
   x. and wherein at least one peptide or peptide variant is selected from the group of HLA-A3 binding peptides
   xi. and wherein at least one peptide or peptide variant is selected from the group of HLA-A24 binding peptides
   xii. and wherein at least one peptide or peptide variant is selected from the group of HLA-A11 binding peptides
   xiii. and wherein at least one peptide or peptide variant is selected from the group of HLA-B35 binding peptides
   xiv. and wherein at least one peptide or peptide variant is selected from the group of HLA-B7 binding peptides
b) and any if the adjuvants mentioned above, such as Montanide ISA 51.

In an embodiment described in the specification the HLA-A1, HLA-A2, HLA-A3, HLA-A24, HLA-A11, HLA-B35 and/or HLA-B7 binding peptide as mentioned here above preferably consist of at the most 15, such as at the most 14, 13, 12, 11, and most preferably at the most 10 amino acids.

In a preferred embodiment described in the specification the vaccine composition comprises the HLA-A1 binding peptide FTELTLGEF (SEQ ID NO: 16), the HLA-A2 binding peptide LMLGEFLKL (SEQ ID NO: 5), the HLA-A3 binding peptide RISTFKNWPK (SEQ ID NO: 20), the HLA-A24 binding peptide STFKNWPFL (SEQ ID NO: 41), the HLA-A11 binding peptide DLAQCFFCFK (SEQ ID NO: 19), the HLA-B35 binding peptide EPDLAQCFY (SEQ ID NO: 9) and the HLA-B7 binding peptide LPPAWQPFL (SEQ ID NO: 10) and an adjuvant such as Montanide ISA 51.

In a further preferred embodiment described in the specification the vaccine composition additionally comprises
i. at least one peptide or peptide variant selected from the group of HLA-B44 binding peptides and/or,
ii. at least one peptide or peptide variant selected from the group of HLA-B27 binding peptides and/or,
iii. at least one peptide or peptide variant selected from the group of HLA-B51 binding peptides.

In a particularly preferred embodiment described in the specification the vaccine composition comprises the HLA-A1 binding peptide FTELTLGEF (SEQ ID NO: 16), the HLA-A2 binding peptide LMLGEFLKL (SEQ ID NO: 5), the HLA-A3 binding peptide RISTFKNWPK (SEQ ID NO: 20), the HLA-A24 binding peptide STFKNWPFL (SEQ ID NO: 41), the HLA-A11 binding peptide DLAQCFFCFK (SEQ ID NO: 19), the HLA-B35 binding peptide EPDLAQCFY (SEQ ID NO: 9) and the HLA-B7 binding peptide LPPAWQPFL (SEQ ID NO: 10) and any on or more the HLA-B44 binding peptide is KETNNKKKEY (SEQ ID NO: 42), the HLA-B27 binding peptide is ERMAEAGFI (SEQ ID NO: 43), and the HLA-B51 binding peptide Is RAIEQLAAM (SEQ ID NO: 44) and an adjuvant such as Montanide ISA 51.

in any of the above embodiments described in the specification, the HLA-A11 binding peptide may be selected from the group of DLAQCFFCFK (SEQ ID NO:19), DVAQCFFCFK (SEQ ID NO: 45), DFAQCFFCFK (SEQ ID NO: 46) or DIAQCFFCFK (SEQ ID NO: 47).

In any of the above embodiments, it is preferred that the number of peptides multiplied by maximally 13, such as 12, such as 11 or preferably 10 or 9, is the most number of amino acids the vaccine consists of. For example, if a vaccine comprises 5 peptides it is thus preferred that vaccine consists of at the most 65, such as 60, such as 55, such as 50 or 45 amino acids.

In order to select survivin peptide or survivin peptide variant for use in the vaccine composition according to the Invention the capability of the survivin peptide and peptide variants for binding a HLA Class one molecule may be evaluated. Furthermore the ability of the survivin peptide or survivin peptide variant to eliciting INF-γ-producing cells in a PBL population of a cancer patient may be evaluated.

These measurements may give an indication of the usefulness of the survivin peptide or survivin peptide variant for use in a vaccine composition, but it is preferred that the vaccine composition comprising survivin peptide or survivin peptide variants are capable of inducing a strong specific T-cell response in a cancer patient upon administration. It is furthermore preferred that administration of the vaccine composition induces a clinical response that is characterised as described in the section related to evaluation of target lesions. The clinical response may be characterised as at least stable disease (an increased of at the most 20 % in the sum of the longest diameter of target lesions) more preferably a decrease in the sum of the longest diameter of target lesions, such a partial response or most preferably complete regression.

The vaccine composition comprises one or more survivin peptide(s) or peptide variant(s), wherein the one or more survivin peptide(s) or peptide variant(s) is/are restricted to a HLA class one molecule, wherein a restricted peptide or peptide variants is characterised by having at least one of the following features;
(i) capable of binding to the Class I HLA molecule with an affinity, as measured by the amount of the peptide that is capable of half maximal recovery of the Class I HLA molecule (C₅₀ value), which is at the most 50 µM as determined by the assembly binding assay as described in WO 2004/067023.
(ii) capable of eliciting INF-y-producing cells in a PBL population of a cancer patient at a frequency of at least 1 per 10⁴ PBLs as determined by an ELISPOT assay (as described in WO 2004/067023).

The assembly binding assay provides a simple means of screening candidate peptides for their ability to bind to a given HLA allele molecule at the above affinity. In preferred embodiments, the one or more survivin peptides or survivin peptide variants have a C₅₀ value, which is at the most 30 µM, such as at the most 20 µM including, such as at the most 10 µM, at the most 5 µM and at the most 2 µM or at the most 1 µM. The C₅₀ values of selected peptides are shown in Table 4 in WO 2004/067023.

A feature of the one or more survivin peptides or survivin peptide variants for use in a vaccine composition according to the invention is/are the capability to recognise or elicit INF-y-producing responder T cells, i.e. cytotoxic T cells (CTLs) that specifically recognise the particular peptide in a PBL population or tumor cells of a cancer patient (target cells). This activity is readily determined by subjecting PBLs or tumor cells from a patient to an ELISPOT assay as described in WO2004/067023 and reference therein. Prior to the assay, it may be advantageous to stimulate the PBL population or the tumor cells to be assayed by contacting the cells with the peptide to be tested. Preferably, the peptide is capable of eliciting or recognising INF-y-producing T cells at a frequency of at least 1 per 10⁴ PBLs as determined by an ELISPOT assay as used herein. More preferably the frequency is at least 5 per 10⁴ PBLs, most preferably at least 10 per 10⁴ PBLs, such as at least 50 or 100 per 10⁴ PBLs. In specific preferred embodiments the frequency is at least 200 per 10⁴ PBLs or such as 250 per 10⁴ PBLs.

IFN-y production in response to melanoma specific antigens, such as MART-1 and gp100 peptides, has been demonstrated upon vaccination of cancer patients with the indicated peptides. But no significant association with clinical responses was observed (Hersey, P. et al., Cancer Immunol. Immunother. 2004, Sep. 21).

Thus, following identification of putative immunogens evaluation of the function in vivo is preferred. It is highly preferred that the vaccine composition comprising one or more survivin peptide(s) or peptide variant(s) capable of eliciting a very strong immunological response, such as an induction of a very strong specific cytotoxic T- cell response as measured by ELISPOT assays for INF-y, before and after vaccination. Such assays involves testing the cytotoxic T- cell response in the patients by analysing PBMCs obtained prior to and after administration of the vaccination composition, for reactivity to the immunogen used in the vaccine composition, by for example an ELISPOT assays as described in example 1.

The use of dendritic cells in vaccine compositions has in the prior art yielded a higher efficiency as compared to oil based adjuvants (Schreurs MW et all, Cancer Res. 2000 Dec 15;60(24):6995-7001). Therefore dendritic cells are presently the preferred adjuvant.

The surprising results of the clinical trial procedure described in example 1 showed that administration of a vaccine composition according to the present invention was capable of inducing a surprisingly high number of specific INF-y releasing cells.

It is preferred that administration of a vaccine composition according to the invention is capable of inducing a strong specific T- cell response in a subject as measured by the number of INF-y releasing cells, that is more than 50 per 10⁴ PBMC cells, or such as more than 100 per 10⁴ PBMC cells, or such as more than 150 per 10⁴ PBMCs, or such as more than 200 per 10⁴ PBMC cells. In is most preferred that the specific T- cell response as measured by the number of INF-y releasing cells is more than 250 per 10⁴ PBMCs.

The specific T- cell response as measured by the ELISPOT may depend upon the administration scheme employed e.g. the number of vaccination and the timing of administering the vaccine composition (see description relating to treatment). In an embodiment a strong specific cytotoxic T-cell response can be detected after 12 months, or such as after 10 months, as after 8 months. In a preferred embodiment the specific cytotoxic T-cell response can be detected after 6 months. In further most preferred embodiment the specific cytotoxic T-cell response can be detected after 4 or 3 months.

It may further be relevant to evaluate the anti-angiogenic effect of the peptides, as inhibition of angiogenesis has a profound effect on the development of solid tumors.

An indicator of a potential anti-angiogenic effect is infiltration of tumor stroma with antigen specific T-cells. The presence of antigen specific T-cells in tumor stroma may be tested using a method of tissue staining as described in example 2, whereby antigen specific T-cells in situ in tumor lesions of cancer patients are detected using multimerised peptide/HLA complexes. The antigen specific T-cells may recognise a survivin peptide or survivin peptide variant according to the invention, preferably in complex with a HLA class 1 molecule.

It is preferred that administration of a vaccine composition according to the invention is capable of inducing infiltration of antigen specific T- cell in the tumor stroma, such as survivin specific T- cells in the tumor stroma.

The evaluation of a vaccine composition further includes examining the capability of the vaccine composition in eliciting a clinical response upon administration. In relation to treatment of cancer the clinical response may be measured using Response Evaluation Criteria in Solid Tumors (RECIST) as described in the section below relating to evaluation of target lesions.

In an embodiment the vaccine composition according to the invention is capable of eliciting a clinical response, referred to as stable disease, partial response or complete regression, characterised by an increased of at the most 20 % in the sum of the longest diameter of target lesions.

Thus, a simple approach to identify peptide variants of potential use in a vaccine composition according to the invention includes the following steps: selecting a particular HLA molecule, e. g. one occurring at a high rate in a given population, carrying out an alignment analysis as described above to identify "anchor residue motifs" in the survivin protein, isolating or constructing peptides of a suitable size that comprise one or more of the identified anchor residues and testing the resulting peptides for (i) capability to bind to the particular HLA molecule using the assembly assay as described herein, and/or (ii) the capability of the peptides to elicit INF-γ-producing cells in a PBL population from a cancer patient at a frequency of at least 1 per 10⁴ PBLs as determined by an ELISPOT assay as described in WO2004/067023.

To establish if the identified peptides or peptide variants are useful in a vaccine composition according to the invention the capability, of the vaccine composition comprising the peptide, of eliciting an immunological response, such as an induction of a strong specific cytotoxic T- cell response may be measured by ELISPOT assays for INF-y before and after vaccination (as describe in example 1 herein.)

In WO2004/067023 it was shown that survivin reactive cells isolated by means of HLA/peptide complexes possess the functional capacity of lysing target cells. Additionally, it was further demonstrated that a dendrite cell vaccine using survivin peptides and survivin peptide variants is/are able to elicit a week immune responses in cancer cell lines and in PBL populations from cancer patients, but the clinical responses in the cancer patients were modest as progressive disease was reported.

The specific immune response may be evaluated relative to the total number of PBMCs or the number of CD8⁺ cells in the population. The latter evaluation reference was used in WO 2004/067023, whereas the result described here in uses the reference of the total number of PBM cells. If the results described in figure 17 of WO 2004/067023 had used the same reference the number of reactive cells would have been 8-30 per 10⁴ PBMC cells.

As the strength of the immune response is a critical parameter in evaluation of potential immunogens, this analysis is a strong tool in evaluating a vaccine composition. According to the invention the capability of stimulating a strong specific T-cell response upon administration to a cancer patient is an important feature. The response may be measured by the number of INF-γ producing cells in a population of PBMCs before and after immunization with the vaccine composition. In an embodiment of the invention the vaccine composition is capable of stimulating a strong specific T-cell response in a cancer patient, wherein a strong T-cell response as measured by ELISPOT assay after administration is more than 50, such as more than 100, such as more than 150, such as more than 200, such as more than 225 or such as more than 250 peptide specific spots per 10⁴ PBL cells.

Evaluation of the anti-angiogenic effect may be used for selection of useful peptides as it is believed, with out being bound by the theory, that a vaccine composition capable of eliciting an anti-angiogenic effect will prove to very effective in inhibition of tumor growth. If combined with peptide(s) capable of eliciting a strong specific T-cell response against survivin, it is expected that vaccine composition comprising such peptide(s) will have a very high probability of inducing a good clinical response.

An aspect of the invention relates to a vaccine composition comprising one or more survivin peptides or peptide variant(s), wherein the sequence of the peptide variant, over the entire length, is at least 85 % identical to a consecutive amino acid sequence of SEQ ID NO: 23, and an adjuvant capable of inducing infiltration of antigen specific T-cells in tumor stroma in a subject.

In a preferred embodiment the vaccine composition is capable of inhibiting angiogenesis in a subject.

It is further essential to evaluate the effect on target lesions, thus the clinical response after administration of the vaccine composition must be evaluated as described below in the section related to evaluation of target lesion. It is preferred that the vaccine composition is capable of eliciting a clinical response, referred to as stable disease, partial response or complete regression, as characterised by an increased of at the most 20 % in the sum of the longest diameter of target lesions as seen in example 1. A decrease in the sum of the longest diameter of target lesions is more preferred (partial response) and a most preferred response is complete remission.

It is contemplated that the survivin peptides or survivin peptide variant in addition to their capacity to bind to HLA molecules, are capable of forming complexes of HLA and peptides on cell surfaces, which complexes in turn act as epitopes or targets for cytolytic T cells. It is possible that the survivin peptides or survivin peptide variants may elicit other types of immune responses, such as B-cell responses resulting in the production of antibodies against the complexes and/or a Delayed Type Hypersensitivity (DTH) reaction.

The latter type of immune response is defined as a redness and palpable induration at the site of injection of the vaccine composition of the invention.

Possible side-effects of immunisation could be systemic or local toxicity. Vascular alterations, such as vasculitis or impaired wound healing are possible side effects. Alterations in hemolgobin, leucocytes and thrombocytes as well as lactate dehydrogenase, creatinine and cholinesterase are other unwanted effects.

In an embodiment of the invention administration of the vaccine composition have no vascular alteration. In a second embodiment administration of the vaccine composition does not induce impaired wound healing.

Thus in a most preferred embodiment of the invention administration of the vaccine composition have essentially no side effect. In particular the relevance of side effects should be evaluated in relation to the severity of the disease.

Furthermore, as previously described, there has been an increased focus on eliciting tumor-specific T helper cell immunity, i.e., vaccinating with class II-MHC restricted epitopes despite the fact that tumors generally do not express class II MHC. This is based on the recent finding that the induction and efficacy of the vaccine-induced antitumor response in many cases requires the cooperation of tumor-specific CD4 positive Th cells.

Thus, an important factor driving the development of vaccines having a more complex composition is the desire to target multiple tumor antigens e. g. by designing vaccines comprising or encoding a collection of carefully selected CTL and Th cell epitopes.

### Multi-epitope vaccines

Obviously, multi-epitope vaccines constitute an efficient way to raise immunity against epitopes derived from several different antigens without the need for introducing (genes encoding) potentially hazardous proteins such as oncoproteins. Such vaccines also permit selective induction of immunity against subdominant and cryptic T cell epitopes, which can be especially important in the case of tumor-associated autoantigens for which tolerance may exist for the epitopes that are prominently presented in normal tissues.

Some problems associated with epitope vaccines include failure of the antigen-presenting cells to present certain epitopes. In particular antigens expressed on tumor cells may be differently presented due to functional differences between the immunoproteasomes of antigen presenting cells and the 'constitutive' proteasomes present in most tumor cells.

Thus the identification of peptides suited for vaccine composition involves testing and selection based on experimental research to evaluate the efficiency of the different compounds that may be included in the vaccine composition, including both the antigen and adjuvant component of the vaccine composition.

Accordingly, in a further aspect the present invention provides a vaccine composition comprising one or more survivin peptide or survivin peptide variants alone or in suitable combination with other proteins or peptide fragments. In specific embodiments such other proteins or peptide fragments include but are not limited to proteins involved in regulation of cell apoptosis or peptide fragments hereof. Suitable examples of such proteins can be selected from the Bcl-2 protein family, e.g., the Bcl-2 protein, the Bcl-X_{L} protein, the Bcl-w protein, the Mcl-1 protein, the TRAG-3 protein and peptide fragments derived from any of the proteins. Other known apoptosis inhibitors include members of the inhibitor of apoptosis protein (IAP) family such as X-IAP, C-IAP1 and C-IAP2 these proteins are all relatively ubiquitously expressed whereas the inhibitor of apoptosis polypeptide ML-IAP has a rather selective expression, and is predominantly detected in melanomas. Thus, fragments of ML-IAP capable of eliciting a specific T-cell response i.e. a cytotoxic T-cell response or a helper T-cell response may optionally be included in the vaccine composition of the present invention.

Useful peptide fragments of ML-IAP include ML-IAP₂₄₅ (RLQEERTCKV) (SEQ ID NO: 24),ML-IAP₂₈₀ (QLCPICRAPV) (SEQ ID NO: 25),ML-IAP₉₀ (RLASFYDWPL) (SEQ ID NO: 26),ML-IAP₁₅₄ (LLRSKGRDFV) (SEQ ID NO: 27), ML-IAP₂₃₀ (VLEPPGARDV) (SEQ ID NO: 28), ML-IAP₉₈ (PLTAEVPPEL) (SEQ ID NO: 29), ML-IAP₃₄ (SLGSPVLGL) (SEQ ID NO: 30), ML-IAP₅₄ (QILGQLRPL) (SEQ ID NO: 31), ML-IAP₉₉ (LTAEVPPEL) (SEQ ID NO: 32),ML-IAP₈₃ (GMGSEELRL) (SEQ ID NO: 33) andML-IAP₂₀₀ (ELPTPRREV) (SEQ ID NO: 34).

Additionally, the pharmaceutical composition of the invention may advantageously comprise at least one further immunogenic protein or peptide fragment hereof selected from a protein or peptide fragment not belonging to or derived from the survivin protein. In specific embodiments, the immunogenic protein or peptide fragment thereof is derived from the Bcl-2 protein family as described above and in PCT/DK2004/000799. A further immunogenic Bcl-2-derived peptide is an HLA-A2 restricted peptide having a sequence selected from the following: Bcl₁₇₂ (NIALWMTEYL) (SEQ ID NO: 35), Bcl₁₈₀ (YLNRHLHTWI) (SEQ ID NO: 36), Bcl₂₀₈ (PLFDFSWLSL) (SEQ ID NO: 37) and Bcl₂₁₄ (WLSLKTLLSL) (SEQ ID NO: 38), Bcl₂₁₈ (KTLLSLALV) (SEQ ID NO: 39) and Bcl₈₀ (AAAGPALSPV) (SEQ ID NO: 40).

An embodiment of the invention relates to a vaccine composition according to invention further comprising one or more peptides or peptide variants selected from the groups of ML-IAP, BCL-2, BCL-X, MCL-1 or TRAG-3 peptides (as described in PCT/DK2004/000798) or peptide variants thereof capable of binding a HLA class 1 molecule.

Additionally, the composition according to the present invention may be provided as a multiepitope vaccine comprising class I restricted epitope and/or class II restricted epitopes as defined hereinbefore.

### Dose

It is contemplated that useful vaccine compositions of the inventions comprise an immunologically effective amount of the survivin peptide or survivin peptide variants.

The amount of the survivin peptide or survivin peptide variants in the vaccine composition may vary, depending on the particular application. However, a single dose of the immunogen is preferably anywhere from about 10 µg to about 5000 µg more preferably from about 25 µg to about 2500 µg, or such as from about 50 µg to about 1000 µg, or such as from about 50 µg to about 500 µg, or such as from about 50 µg to about 250 µg, or such as from about 50 µg to about 200 µg, or such as from about 75 µg to about 150 µg. In a preferred embodiment a dose of the immunogen is from about 75 µg to about 150 µg. In a most preferred embodiment a dose is about 100 µg.

### Administration

Modes of administration include intradermal, subcutaneous and intravenous administration, implantation in the form of a time release formulation, etc. Any and all forms of administration known to the art are encompassed herein. Subcutaneous administration is preferred and in particular deep subcutaneous administration. It is further preferred that the vaccine composition according to the invention is administered into alternating extremities in close vicinity of the draining lymph node.

Also any and all conventional dosage forms that are known in the art to be appropriate for formulating injectable immunogenic peptide composition are encompassed, such as lyophilised forms and solutions, suspensions or emulsion forms containing, if required, conventional pharmaceutically acceptable carriers, diluents, preservatives, adjuvants, buffer components, etc.

The immunologic effect of the composition of the invention can be determined using several approaches as know by a person skilled in the art and as described in the examples of WO 2004067023. An example on how to determine a CTL response provoked by the vaccine composition is provided in WO97/28816. A successful immune response may also be determined by the occurrence of Delayed Type Hypersensitivity (DTH) reactions after immunisation and/or the detection of antibodies specifically recognising the peptide (s) of the vaccine composition.

In preferred embodiments, the pharmaceutical composition of the invention is an immunogenic composition or vaccine capable of eliciting an immune response to a cancer disease.

As used herein, the expression "immunogenic composition or vaccine" refers to a composition eliciting at least one type of immune response directed against cancer cells. Thus, such an immune response may be any of the types mentioned above: A CTL response where CTLs are generated that are capable of recognising the HLA/peptide complex presented on cell surfaces resulting in cell lysis, i. e. the vaccine elicits the production in the vaccinated subject of effector T-cells having a cytotoxic effect against the cancer cells; a B-cell response giving rise to the production of anti-cancer antibodies; and/or a DTH type of immune response.

### Nucleic acid vaccines

The vaccine composition according to the present specification may comprise a nucleic acid encoding the survivin polypeptide (SEQ ID NO: 23), a peptide fragment thereof or a survivin peptide variant thereof. Said nucleic acid may thus encode any of the above-mentioned protein and peptide fragments. The nucleic acid may for example be DNA, RNA, LNA, HNA, PNA, preferably the nucleic acid Is DNA or RNA.

In an embodiment the specification describes a vaccine composition comprising:
i. a nucleic add encoding:
   a) the survivin polypeptide (SEQ ID NO: 23),
   b) a survivin peptide or
   c) a survivin peptide variant
      and
ii. any of the adjuvant mentioned above.

The nucleic acids of the invention may be comprised within any suitable vector, such as an expression vector. Numerous vectors are available and the skilled person will be able to select a useful vector for the specific purpose. The vector may, for example, be in the form of a plasmid, cosmid, viral particle or artificial chromosome. The appropriate nucleic acid sequence may be inserted into the vector by a variety of procedures, for example, DNA may be Inserted into an appropriate restriction endonuclease site(s) to using techniques well known in the art. Apart from the nucleic acid sequence according to the invention, the vector may furthermore comprise one or more of a signal sequence, an origin of replication, one or more marker genes, an enhancer element, a promoter, and a transcription termination sequence. The vector may also comprise additional sequences. Construction of suitable vectors containing one or more of these components employs standard ligation techniques which are known to a person skilled in the art. The vector is preferably an expression vector, comprising the nucleic acid operably linked to a regulatory nucleic acid sequence directing expression thereof in a suitable cell. Within the scope of the present invention said regulatory nucleic acid sequence should in general be capable of directing expression in a mammalian cell, preferably a human cell, more preferably in an antigen presenting cell.

In one preferred embodiment the vector is a viral vector. Said viral vector may in addition to the nucleic acid encoding survivin or peptide fragment thereof comprise a second nucleic acid sequence encoding a T-cell stimulatory polypeptide. The T-cell stimulatory polypeptide is preferably selected from the group consisting of B7.1, ICAM-1 and LFA-3.

The vector may also be a bacterial vector, such as an attenuated bacterial vector. Attenuated bacterial vectors may be used in order to induce lasting mucosal immune responses at the sites of infection and persistence. Different recombinant bacteria may be used as vectors, for example the bacterial vector may be selected from the group consisting of *Salmonella*, Lactococcus], and Listeria. In general, induction of immunity to the heterologous antigen HPV16 L1 or E7 could be shown, with strong CTL induction and tumor regression in mice.

### Pharmaceutical medicament

An aspect of the invention relates to the vaccine compositions according to the invention for use in the manufacture of a medicament. In a specific embodiment the medicament is for the treatment of cancer diseases.

In an embodiment the medicament according to the invention is for subcutaneous administration, thus the medicament may be formulated as a solution or suspension or alternatively as a freeze dried product for suspension prior to administration.

The invention further relates to a medicament for treating a cancer comprising a vaccine composition comprising one or more survivin peptide or survivin peptide variants and an adjuvant as an active ingredient.

### Treatment

The survivin molecule has been found to be deregulated in a large population of cancer diseases. A vaccine composition comprising the survivin peptide or survivin peptide variants may be used for treatment of clinical conditions such as cancer diseases. According to the invention treatment include decreasing symptoms and inhibiting progression of the disease, thus resulting in a clinical response as described here below.

The vaccine composition according to the invention may be administered more than once, such as twice, three times, four times, five times, or such as more than five time, such as more than 7 times, such as more than 10 times, such as more than 15 times. The disease treated using the vaccine composition of the invention may be recurring or chronic, thus in order to minimize symptoms and inhibit progression or reoccurrence of the disease the treatment may be continued with regular intervals for a prolonged period. For example, the disease treated may be a cancer disease and the treatment may be continued until or as long as complete regression or stable disease is the clinical response, or for the life of the patient.

The vaccine composition may be administered such as once every 14 day, for at least a month, such as at least two months, such as at least 3 months, such as at least 5 months, such as at least 8 months, such as at least 12 months, such as at least 20 months. The vaccine composition may be administered with regular intervals for the life of the subject. The vaccine composition may be administered when the disease reoccurs or when progression of the disease is detected.

In an embodiment the invention relates to a method of stimulating a strong specific T-cell response against survivin in a subject, said method comprising:
a) providing a vaccine composition according to the invention,
b) administering said vaccine composition to the subject, wherein said vaccine composition may be administered more than once; and
c) thereby stimulating a strong specific T-cell response in the subject, wherein the specific T-cell response, when measured by ELISPOT assay before and after administration of the vaccine composition, is more than 50 peptide specific spots per 10⁴ PBMC cells.

In a specific embodiment the strong specific T-cell response, when measured by ELISPOT assay before and after administration of the vaccine composition, is more than 200 peptide specific spots per 10⁴ PBMC cells.

In a further specific embodiment the method according to the invention may be include administering said vaccine composition once every month.

In a different preferred embodiment the vaccine composition is administered once every second month.

In an embodiment the invention relates to a method of treatment or preventing a disease comprising;
a) providing a vaccine composition comprising any of the above mentioned peptides and optionally any of the above mentioned adjuvants,
b) administering said vaccine composition to the subject, wherein said vaccine composition may be administered more than once; and
c) thereby stimulating a strong specific T-cell response in the subject, wherein the strong specific T-cell response, when measured by ELISPOT assay, before and after administration of the vaccine composition, is more than 50 peptide specific spots per 10⁴ PBMC cells.
d) obtaining a clinical response in the subject.

The clinical response is evaluated as described in the section concerning evaluation of target lesions below.

In a preferred embodiment the vaccine composition is for the treatment of a clinical condition.

In a preferred embodiment of the invention, the clinical condition is a cancer. The term "cancer" as used herein is meant to encompass any cancer, neoplastic and preneoplastic disease. Said cancer may for example be selected from the group consisting of; colon carcinoma, breast cancer, pancreatic cancer, ovarian cancer, prostate cancer, fibrosarcoma, myxosarcoma, liposarcoma, chondrosarcoma, osteogenic sarcoma, chordoma, angiosarcoma, endotheliosarcoma, lymphangeosarcoma, lymphangeoendothelia sarcoma, synovioma, mesothelioma, Ewing's sarcoma, leiomyosarcoma, rhabdomyosarcoma, squamous cell carcinoma, basal cell carcinoma, adenocarcinoma, sweat gland carcinoma, sebaceous gland carcinoma, papillary carcinoma, papillary adenocarcinomas, cystandeocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioblastomas, neuronomas, craniopharingiomas, schwannomas, glioma, astrocytoma, medulloblastoma, craniopharyngioma, ependymoma, pinealoma, hemangioblastoma, acoustic neuroama, oligodendroglioma, meningioma, melanoma, neuroblastoma, retinoblastoma, leukemias and lymphomas, acute lymphocytic leukemia and acute myelocytic polycythemia vera, multiple myeloma, Waldenstrom's macroglobulinemia, and heavy chain disease, acute nonlymphocytic leukemias, chronic lymphocytic leukemia, chronic myelogenous leukemia, Hodgkin's Disease, non-Hodgkin's lymphomas, rectum cancer, urinary cancers, uterine cancers, oral cancers, skin cancers, stomach cancer, brain tumors, liver cancer, laryngeal cancer, esophageal cancer, mammary tumors, childhood-null acute lymphoid leukemia (ALL), thymic ALL, B-cell ALL, acute myeloid leukemia, myelomonocytoid leukemia, acute megakaryocytoid leukemia, Burkitt's lymphoma, acute myeloid leukemia, chronic myeloid leukemia, and T cell leukemia, small and large non-small cell lung carcinoma, acute granulocytic leukemia, germ cell tumors, endometrial cancer, gastric cancer, cancer of the head and neck, chronic lymphoid leukemia, hairy cell leukemia and thyroid cancer.

In an embodiment the vaccine composition according to the invention is for the treatment of a cancer selected from the group of; malignant melanoma, pancreatic cancer, cervix cancer or colon cancer.

In a preferred embodiment the vaccine composition according to the invention is for the treatment of malignant melanoma and pancreatic cancer.

An aspect of the invention relates to a method of inhibiting angiogenesis comprising;
a) providing a vaccine composition according to the invention
b) administering said vaccine composition to a subject.

The individual in need of treatment may be any individual, preferably a human being. Peptides will in general have different affinities to different HLA molecules. Hence, in the embodiments of the present invention wherein the vaccine composition or the pharmaceutical composition comprises survivin peptides, it is preferred that a vaccine composition or a pharmaceutical composition to be administered to a given individual will comprise at least one peptide capable of associating with HLA molecules of that particular individual.

### Combination therapy

The present invention furthermore relates to pharmaceutical compositions and kit-of-parts for use in combination therapy.

Combination therapy as used herein denotes treatment of a subject in need thereof with more than one different medicament. Hence combination therapy may in one aspect involve administration of a pharmaceutical compositions or a kit of parts comprising a vaccine composition as described herein above and for a secondary medicament. The secondary medicaments may be any of the medicaments described herein below, for example a chemotherapeutic agent or inhibitors of angiogenesis.

In particular combination therapy may involve administration to an individual of a chemotherapeutic agent and/or an immunotherapeutic agent in combination with one or more of i) the survivin peptides or survivin peptides variants according to the invention, ii) vaccine compositions according to the invention. However, combination therapy may also involve radiation therapy, gene therapy and/or surgery.

An aspect of the invention relates to a method of combination therapy including simultaneously, sequentially or separate administration in any order, of:
i) a vaccine composition according to the invention
ii) and a secondary medicament.

In a preferred embodiment the secondary medicament is a chemotherapeutic agent.

The chemotherapeutic agent can be e.g. methotrexate, vincristine, adriamycin, cisplatin, non-sugar containing chloroethylnitrosoureas, 5-fluorouracil, mitomycin C, bleomycin, doxorubicin, dacarbazine, taxol, fragyline, Meglamine GLA, valrubicin, carmustaine and poliferposan, MM1270, BAY 12-9566, RAS famesyl transferase inhibitor, famesyl transferase inhibitor, MMP, MTA/LY231514, LY264618/Lometexol, Glamolec, CI-994, TNP-470, Hycamtin/Topotecan, PKC412, Valspodar/PSC833, Novantrone/Mitroxantrone, Metaret/Suramin, Batimastat, E7070, BCH-4556, CS-682, 9-AC, AG3340, AG3433, Incel/VX-710, VX-853, ZD0101, ISI641, ODN 698, TA 2516/Marmistat, BB2516/Marmistat, CDP 845, D2163, PD183805, DX8951f, Lemonal DP 2202, FK 317, Picibanil/OK-432, AD 32/Valrubicin, Metastron/strontium derivative, Temodal/Temozolomide, Evacet/liposomal doxorubicin, Yewtaxan/Placlitaxel, Taxol/Paclitaxel, Xeload/Capecitabine, Furtulon/Doxifluridine, Cyclopax/oral paclitaxel, Oral Taxoid, SPU-077/Cisplatin, HMR 1275/Flavopiridol, CP-358 (774)/EGFR, CP-609 (754)/RAS oncogene inhibitor, BMS-182751/oral platinum, UFT(Tegafur/Uracil), Ergamisol/Levamisole, Eniluracil/776C85/5FU enhancer, Campto/Levamisole, Camptosar/Irinotecan, Tumodex/Ralitrexed, Leustatin/Cladribine, Paxex/Paclitaxel, Doxil/liposomal doxorubicin, Caelyx/liposomal doxorubicin, Fludara/Fludarabine, Pharmarubicin/Epirubicin, DepoCyt, ZD1839, LU 79553/Bis-Naphtalimide, LU 103793/Dolastain, Caetyx/liposomal doxorubicin, Gemzar/Gemcitabine, ZD 0473/Anormed, YM 116, Iodine seeds, CDK4 and CDK2 inhibitors, PARP inhibitors, D4809/Dexifosamide, Ifes/Mesnex/Ifosamide, Vumon/Teniposide, Paraplatin/Carboplatin, Plantinol/cisplatin, Vepeside/Etoposide, ZD 9331, Taxotere/Docetaxel, prodrug of guanine arabinoside, Taxane Analog, nitrosoureas, alkylating agents such as melphelan and cyclophosphamide, Aminoglutethimide, Asparaginase, Busulfan, Carboplatin, Chlorombucil, Cytarabine HCl, Dactinomycin, Daunorubicin HCl, Estramustine phosphate sodium, Etoposide (VP16-213), Floxuridine, Fluorouracil (5-FU), Flutamide, Hydroxyurea (hydroxycarbamide), Ifosfamide, Interferon Alfa-2a, Alfa-2b, Leuprolide acetate (LHRH-releasing factor analogue), Lomustine (CCNU), Mechlorethamine HCl (nitrogen mustard), Mercaptopurine, Mesna, Mitotane (o.p'-DDD), Mitoxantrone HCl, Octreotide, Plicamycin, Procarbazine HCl, Streptozocin, Tamoxifen citrate, Thioguanine, Thiotepa, Vinblastine sulfate, Amsacrine (m-AMSA), Azacitidine, Erthropoietin, Hexamethylmelamine (HMM), Interleukin 2, Mitoguazone (methyl-GAG; methyl glyoxal bis-guanylhydrazone; MGBG), Pentostatin (2'deoxycoformycin), Semustine (methyl-CCNU), Teniposide (VM-26) and Vindesine sulfate. Furthermore, the chemotheraputic agent may be any of the chemotherapeutic agents mentioned in table 3 of US 6,482,843 columns 13 to 18.

The therapeutic compositions or vaccine compositions of the invention can also be used in combination with other anti-cancer strategies, and such combination therapies are effective in inhibiting and/or eliminating tumor growth and metastasis. The methods of the present invention can advantageously be used with other treatment modalities, including, without limitation, radiation, surgery, gene therapy and chemotherapy.

Survivin is highly expressed in endothelial cells during angiogenesis and may be implicated in the cytoprotective effect of vascular endothelia growth factor (VEGF), thus targeting survivin expressing cells may target cancer cells directly and further prevent tumor growth by inhibition of angiogenesis.

The anti-angiogenic effect may be enhanced by combining treatment with a vaccine according to the invention with treatment with angiogenesis inhibitors. Anti-angiogenic therapy targets the tumor vasculature and prevents tumor growth beyond a certain size, thus in a second preferred embodiment the secondary medicament is an inhibitor of angiogenesis.

The inhibitor of angiogenesis may be, but are not limited to, e.g. BMS-275291, Dalteparin (Fragmin®), Suramin, 2-methoxyestradiol (2-ME), Thalidomide, CC-5013 (Thalidomide Analog), Combretastatin A4 Phosphate, LY317615 (Protein Kinase C Beta Inhibitor), Soy Isoflavone (Genistein; Soy Protein Isolate), AE-941 (Neovastat™; GW786034), Anti-VEGF Antibody (Bevacizumab; Avastin™), Interferon-alpha, PTK787/ZK 222584, VEGF-Trap, ZD6474, EMD 121974, Carboxyamidotriazole (CAI), Celecoxib (Celebrex®), Halofuginone Hydrobromide (Tempostatin™), AdPEDF, Macugen, tryptophanyl-tRNA synthetase (TrpRS), rhufab V2 (aka lucentis), squalamine, Retaane 15 mg (anecortave acetate with depot suspension) and Interleukin-12.

"Combination therapy" can include the introduction of heterologous nucleic acids into suitable cells, generally known as gene therapy. For example gene therapy may involve introduction of tumor suppressor genes or apoptosis promoting genes into tumor cells. Alternatively, nucleic acid sequences inhibiting expression of oncogenes or apoptosis inhibiting genes may be introduced to tumor cells. Furthermore, genes that encode enzymes capable of conferring to tumor cells sensitivity to chemotherapeutic agents may be introduced. Accordingly, the present invention in one embodiment provides a method comprising the step of treating cancer by introducing a gene vector, encoding a protein capable of enzymatic conversion of a pro-drug, i.e., a non-toxic compound, into a toxic compound. In the method of the present invention, the therapeutic nucleic acid sequence is a nucleic acid coding for a product, wherein the product causes cell death by itself or in the presence of other drugs. A representative example of such a therapeutic nucleic acid is one, which codes for thymidine kinase of herpes simplex virus. Additional examples are thymidine kinase of varicella zoster virus and the bacterial gene cytosine deaminase, which can convert 5-fluorocytosine to the highly toxic compound 5-fluorouracil.

### Evaluation of target lesions

Response of treatment is measured using RECIST (Response Evaluation Criteria in Solid Tumors) criteria describe in the original WHO Handbook for reporting results of cancer treatment (World Health Organization Offset Publication No 48; 1979) taking into account the measurement of the longest diameter of target lesions (Therasse P et al. J. Natl. Cancer Inst. 2000 Feb 2;92(3): 205-16). The response is divided in; complete response, partial response, progressive disease and stable disease. A complete response is the disappearance of all target lesions, whereas a partial response refers to at least 30 % decrease in the sum of the longest diameter of target lesions. Progressive disease represents an at least 20 % increase in the sum of the longest diameter of target lesions. Stable disease refers to situation where none of the above applies. The duration of the complete response or partial response should be measured from the time where the measurement criteria are first met until the first date that recurrent or progressive disease is documented. The effect is preferably observed in at least one patient.

A partial response refers to a response wherein at least a 30 % decrease in the sum of the longest diameter of target lesions is observed. The response may be further subgroup, whereby a partial response of 40 %, or a partial response of 50 %, or a partial response of 60 %, or a partial response of 70 %, or a partial response of 80 %, or a partial response of 90 %, relates to a treatment where a decrease in the sum of the longest diameter of target lesions of at least 40 %, 50%, 60%, 70%, 80% or 90 %, respectively, has been observed.

In an embodiment the vaccine composition according to the invention is for treatment of a cancer, whereby a partial response of at least 30 % is obtained.

In an embodiment the method including administering the vaccine composition according to the invention is for the treatment of a cancer, and whereby a partial response of at least 40 %, such as at least 50%, such as at least 60%, such as at least 70%, such as at least 80% or such as at least 90 % is obtained.

Stable disease refers to responses where the sum of the longest diameter of target lesions is decreased by 30 % or less and further includes responses wherein the sum of the longest diameter of target lesions is increased by 20 % or less. This type of responses may be subdivided in responses where the sum of the longest diameter of target lesions is decreased by 30 % or less, such as 25 % or less, such as 20 % or less, such as 15 % or less, such as 10 % or such as 5 % or less and in subgroups where the sum of the longest diameter of target lesions is increased by 20 % or less, such as 15 % or less, such as 10 % or such as 5 % or less. Further included are the subgroups wherein the sum of the longest diameter of target lesions is either decrease or increase by at the most 1%, such as 3% or such as at the most 5 %.

Progressive disease represents an at least 20 % increase in the sum of the longest diameter of target lesions. This type or response may be subdivided in responses where the sum of the longest diameter of target lesions is increased by at the most 25 % such as at the most 30 %, such as at the most 35 %, such as at the most 40 %, such as at the most 45 % or such as at the most 50 %. According to the invention a treatment leading to a progressive disease response where the sum of the longest diameter of target lesions is increased by at the most 30 % may be considered a positive result if the diagnosis predict an increase of 50 %. Thus, specific embodiments include treatments where the response is characterised as progressive disease responses and where the sum of the longest diameter of target lesions is increased by at the most 25 % such as at the most 30 %, such as at the most 35 %, such as at the most 40 %, such as at the most 45 % or such as at the most 50 %.

An embodiment of the invention relates to a method of treatment or preventing a disease comprising;
a) providing a vaccine composition according to the invention,
b) administering said vaccine composition to a subject
c) for the treatment of a cancer, and
d) wherein administration of said vaccine composition results in stable disease, partial response or complete regression, characterised by an increased of at the most 20 % in the sum of the longest diameter of target lesions.

The data described in example 1 demonstrate that even in heavily pre-treated patients with far-advanced diseases, extremely strong survivin-specific T cell responses were mounted within the pool of circulating lymphocytes in all the patients examined. As seen in Example 1, Table 3, two subjects, JUSC and OTSC experienced complete regression, whereas stable disease was observed in some cases and a partial response was seen in one subject.

### Kit of parts

Combination treatment involves separate, sequential or simultaneous administration of two or more active ingredients, formulated in one or more medicaments. For convenient usage said medicaments may be included in a single combined product or kit of parts.

An aspect of the invention relates to a kit of parts comprising:
a) a vaccine composition comprising
   i. one or more survivin peptide or survivin peptide variants, wherein the sequence of the peptide variant, over the entire length, is at least 85 % identical to a consecutive amino acid sequence of SEQ ID NO: 23,
   ii. and an adjuvant as described herein,
b) and a secondary medicament.

For the treatment of cancer disease a kit of parts according to the invention the medicament may be a chemotherapeutic agent. In relation to treatment of immune disease the medicament may be an immunotherapeutic agent.

In an embodiment the kit in parts according to invention the medicament comprises a chemotherapeutic agent or an immunotherapeutic agent. In a preferred embodiment the medicament comprises a chemotherapeutic. According to the invention the vaccine composition and the medicament comprised by the kit of parts are for separate, sequential or simultaneous administration. The kit of parts may further comprise and note including information of appropriate usage/administration/dosage of the comprised medicament and vaccine composition.

### Description of Figures

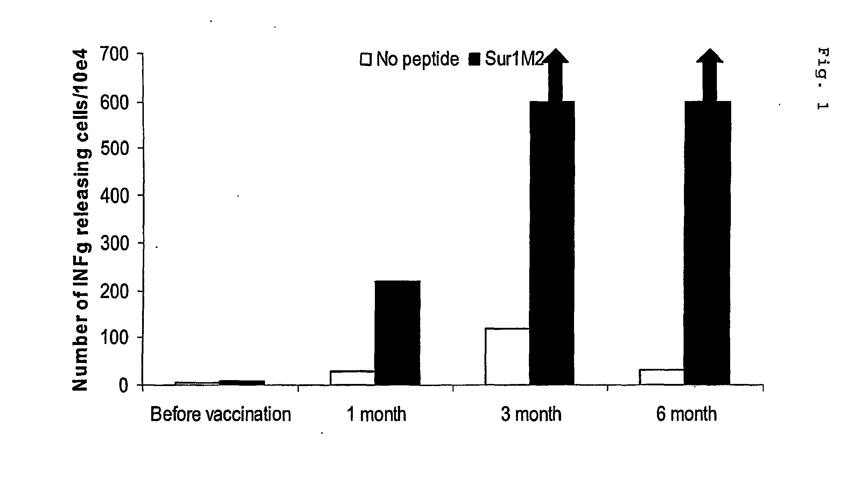

**Figure 1****. Kinetic analysis of immunity in PBL from a pancreatic cancer patient to the survivin peptide Sur1M2 assessed by IFN-γ ELISPOT.** PBMCs from patient OTSC were obtained before the first sur1 M2/Montanide vaccination and one, three and six months thereafter. T-lymphocytes were stimulated once with peptide before plated at 10⁵ cells per well in triplicates either without or with peptide. The average number of peptide specific spots (after subtraction of spots without added peptide) was calculated for each patient using the ImmunoSpot® Series 2.0 Analyzer (CTL Analyzers, LLC, Cleveland, US).

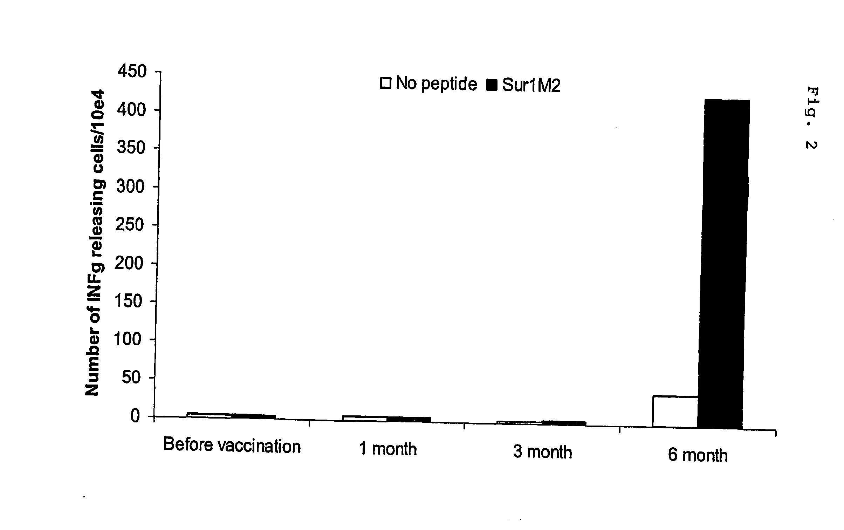

**Figure 2****. Kinetic analysis of immunity in PBL from a melanoma patient to the survivin peptide Sur1M2 assessed by IFN-γ ELISPOT.** PBMCs from patient JUSC were obtained before the first sur1 M2/Montanide vaccination and one, three and six months thereafter. T-lymphocytes were stimulated once with peptide before plated at 10⁵ cells per well in triplicates either without or with peptide. The average number of peptide specific spots (after subtraction of spots without added peptide) was calculated for each patient using the ImmunoSpot® Series 2.0 Analyzer (CTL Analyzers, LLC, Cleveland, US).

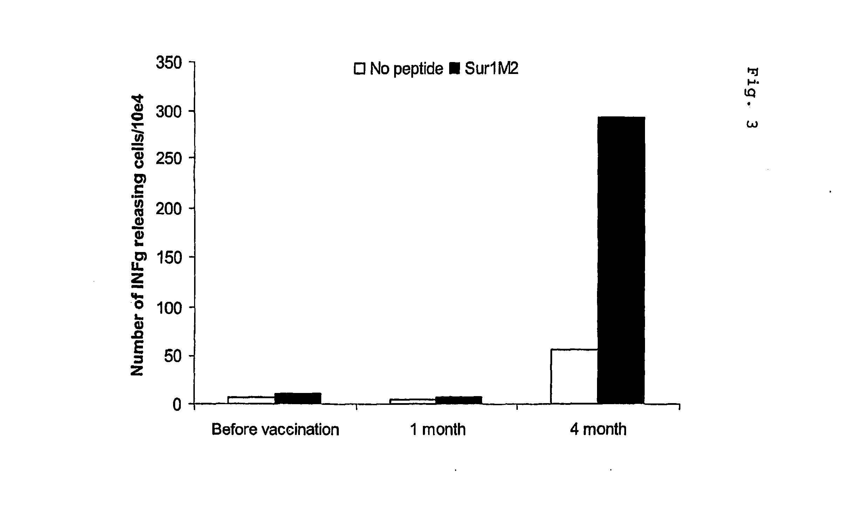

**Figure 3****. Kinetic analysis of immunity in PBL from a melanoma patient to the survivin peptide Sur1M2 assessed by IFN-γ ELISPOT.** PBMCs from patient SIST were obtained before the first sur1M2/Montanide vaccination and one and four months thereafter. T-lymphocytes were stimulated once with peptide before plated at 10⁵ cells per well in triplicates either without or with peptide. The average number of peptide specific spots (after subtraction of spots without added peptide) was calculated for each patient using the ImmunoSpot® Series 2.0 Analyzer (CTL Analyzers, LLC, Cleveland, US).

### Examples

### Example 1

Clinical results using vaccine compositions comprising survivin derived epitopes and montanide ISA 51 as adjuvant. The treatments were performed in a series of late stage cancer patients as described here below.

All clinical procedures were in accordance with the Declaration of Helsinki and all patients provided informed consent prior to therapy. The clinical study was approved by the Ethical review Boards of the University of Würzburg, Germany (Studien-Nr. 7/03) and the Paul-Ehrlich-Institute, Langen, Germany (Vorlagen-Nr 0899/01).

### Patients

To be eligible to participate in this study the patients had to fulfil the following criteria:
- measurable metastatic melanoma, pancreatic, colon or cervical cancer
- confirmed progressive disease
- failure of at least one standard therapy
- life expectancy of at least 3 months
- no therapy within the past 4 weeks
- no gross organ failure
- the class I tissue type HLA-A1, -A2 or -B35

### Peptides

The peptides included in this study were all survivin peptide variants obtained by modification of survivin peptides by substitution of one amino acid. Thereby better anchor residues and improved binding affinity of the given peptide to the MHC molecule was obtained. The peptides used include:
- a HLA-A1 restricted epitope FTELTLGEF (SEQ ID NO: 16)(survivin₉₃₋₁₀₁2T, in which the native glutamine at position 2 was replaced with threonine).
- a HLA-A2 restricted epitope, LMLGEFLKL (SEQ ID NO: 5)(survivin₉₆₋₁₀₄2M, in which the native threonine at position 2 was replaced with methionine).
- a HLA-B35 restricted epitope EPDLAQCFY (SEQ ID NO: 9)(survivin₅₁₋₅₉9Y in which leucine at position 9 was replaced with tyrosine).

One hundred µg of the HLA-A1, HLA-A2 or HLA-B35 restricted survivin peptide was mixed with 1 ml of Montanide ISA51 according to the manufactures (Seppic, Brussels, Belgium) instructions. The mixture was administered by deep subcutaneous injection into alternating extremities in close vicinity of the draining lymph node. Patients were vaccinated at 7-day intervals for the first two vaccinations followed by 28-day intervals for further vaccinations.

### Toxicity, clinical efficacy and immunological responses were assessed.

Toxicity was assessed by physical examination/medical history, haematological tests and serum chemistry. These examinations were performed prior to each vaccination. Clinical efficacy was assessed by physical examination and appropriate imaging studies (such as CT scan, NMR scan, chest X-ray, ultrasound or bone szintigraphy) prior to initiation of therapy and every 3 months thereafter.

Immunological responses were monitored by ELISPOT assay, using PBMCs to detect survivin96-104 specific IFN-γ release. To extend the sensitivity of the ELISPOT assay, PBMCs were stimulated once *in vitro* at a concentration of 1 × 10⁶ cells per ml in 24-well plates (Nunc, Denmark) in X-vivo medium (Bio Whittaker, Walkersville, Maryland), supplemented with 5% heat-inactivated human serum and 2 mM of L-glutamine in the presence of 10 µM of peptide. Two days later, 40 IU/ml recombinant interleukin-2 (IL-2) (Chiron, Ratingen, Germany) were added. After 10 days the cells were tested for reactivity. Briefly, nitrocellulose bottomed 96-well plates (MultiScreen MAIP N45, Millipore, Hedehusene, Denmark) were coated with anti-IFN-γ antibody (1-D1 K, Mabtech, Nacka, Sweden). The wells were washed, blocked by X-vivo medium before adding 10⁴ stimulator T2 cells (with or without 10µM peptide) and effector cells at different concentrations. The plates were incubated overnight. The following day, medium was discarded and the wells were washed prior to addition of biotinylated secondary antibody (7-B6-1-Biotin, Mabtech). The plates were incubated for 2 hours, washed and Avidin-enzyme conjugate (AP-Avidin, Calbiochem, Life Technologies) was added to each well. Plates were incubated at RT for 1 hour and the enzyme substrate NBT/BCIP (Gibco, Life Technologies) was added to each well and incubated at RT for 5-10 min. The reaction was terminated by washing with tap-water upon the emergency of dark purple spots. The spots were counted using the ImmunoSpot® Series 2.0 Analyzer (CTL Analyzers, LLC, Cleveland, US) and the peptide specific CTL frequency could be calculated from the numbers of spot-forming cells. All assays were performed in triplicates for each peptide antigen.

### Results

An overview of the clinical results obtained using survivin peptides including; Patient abbreviations, number of vaccinations and the clinical response is summarised in Table 3.

**Table 3. Results of on-going clinical trail.**

| **Patient** | **Date of birth** | **Diagnosis** | **HLA-type** | **No. of vacc.** | **Ongoing study** | **End date** | **Exits** | **Clinical response** |
|---|---|---|---|---|---|---|---|---|
| AGSC | 28.05.26 | MM | A2 | 2 | X | | | |
| ALKA | 19.04.36 | MM | | 5 | | 22.10.03 | x | |
| BRHI | 27.08.34 | MM | A2 | 5 | X | | | SD |
| CHPF | 18.12.34 | MM | A2 | 12 | X | | | PR |
| CHPF | 18.12.34 | MM | B35 | 1 | X | | | |
| EREI | 04.05.55 | MM | A2 | 5 | | 08.10.03 | X | |
| HAKO | 27.02.63 | MM | A1 | 2 | X | | | |
| JUSC | 03.01.57 | MM | A2 | 16 | X | | | CR |
| MAKR | 20.08.40 | MM | B35 | 3 | X | | | |
| OSRO | 11.07.35 | MM | A2 | 4 | | 05.08.04 | | |
| OTSC | 10.05.29 | PC | A2 | 15 | X | | | CR |
| REPA | 27.09.45 | MM | A2 | 4 | | 19.04.04 | | |
| SIST | 08.02.34 | MM | A2 | 7 | | 30.03.04 | | SD |
| WESE | 23.07.42 | MM | A2 | 5 | X | | | SD |

| | | | | | | | | |
|---|---|---|---|---|---|---|---|---|
| Metastatic melanoma (MM), Pancreatic cancer (PC). Complete response (CR), Partiel response (PR), Stable disease (SD) and Progressive disease (PD). | | | | | | | | |

Toxicity: No therapy-induced side effects were observed. No signs of systemic or local toxicity were observed at the injection sites. Special attention was directed to signs of vascular alteration, e.g. vasculitis or impaired wound healing. Hemoglobin, leucocytes and thrombocytes, as well as lactate dehydrogenase, creatinine and cholinesterase were not influenced by the vaccination therapy (data not shown). Thus, neither clinical nor histological signs for vascular alterations were detectable. Furthermore, there was no sign of impaired wound healing, hemorrhagic disorders, cardiac dysfunction, vasculitis or inflammatory bowel disease. Thus survivin vaccination is both tolerable and safe for cancer patients who retain normal hematopoiesis.

Clinical efficacy: Objective clinical responses were present in this group of patient with rather unfavourable prognosis. The responses included complete tumor regression of visceral metastases in a few patients, but mostly consisted of disease stabilization. Remarkably, in two patients diagnosed with cutaneous melanoma (JuSc) and adeno-carcinoma of the pancreas (OtSc), respectively, complete tumor regression occurred (Table 3) despite the fact that the patients had failed to respond to prior chemotherapy (Temodal® or Gemzar®, respectively). Thus the clinical results demonstrate a very success full treatment with an unusually high response rate and clinical efficacy.

### Survivin-specific CD8+ T cell responses.

The kinetics of the cytotoxic T cell responses was followed in the patiens. PBMCs obtained prior to and after vaccination were tested for reactivity to the modified survivin96-104 epitope by ELISPOT for IFN-γ. In all patients tested, an induction of survivin reactive T cells was evident. For the two complete responding patients OtSc (suffering from pancreatic cancer) and JuSc (suffering from melanoma), PBL were analyzed before vaccination and one, three and six month after the initiation of the vaccinations. In patient OtSc a strong response was present already one month after the initiation of the vaccination trial. This response was even stronger after three and six month. Thus, more than 600 survivin specific cells per 10⁴ could be detected (figure 1). In patient JuSc the response were not detected until after six months (figure 2) however, at this time it was just as strong as in patient OtSc. In addition, we analyzed PBL from a melanoma patient SiSt before vaccination and one and four months after the initiation of the vaccination trial. In patients SiSt a strong response was detected after four months of vaccinations (figure 3). Finally, PBL from two very late stage melanoma patients (AlKa and ErEi) were analyzed, where the patients had only been able to receive four vaccinations before they died of their disease. In both these patients a response against the survivin peptide was introduced (data not shown). The data demonstrates, that even in these heavily pre-treated patients with far-advanced disease, an extremely strong survivin specific T cell responses was mounted within the pool of circulating lymphocytes in all the patients examined. Thus, after *one in vitro* stimulation of PBL obtained from vaccinated patients more than 250 INF-γ releasing cells and in specific examples more than 600 INF-γ releasing cells per 10⁴ cells were detected.

### Example 2

### Immunohistochemistry staining

Biotinylated peptide/HLA complexes are multimerised with streptavidin-FITC-conjugated dextran molecules (DAKO, Glostrup, Denmark) to generate multivalent HLA-dextran compounds for immunohistochemistry. Tissue sections are dried overnight and subsequently fixed in cold acetone for 5 min. All incubation steps are performed in the dark at room temperature: (a) 45 min of the primary antibody (1:100 diluted) (b) Cy 3-conjugated goat antimouse (1:500 diluted; code 115-165-100; Jackson ImmunoResearch, obtained from Dianova, Hamburg, Germany) for 45 min; and finally (c) the multimers for 75 min. Between each step, the slides are washed two times for 10 min in PBS/BSA 0.1 %. The slides are mounted in vectashield and kept in the refrigerator until examination under the confocal microscope (Leica).

## Claims

1. A vaccine composition comprising,
i. one or more HLA-A2 restricted survivin peptide selected from the group consisting of LLLGEFLKL (SEQ ID NO. 4) and LMLGEFLKL (SEQ ID NO 5), and
ii. an adjuvant formulated for a water in oil emulsion comprising a mineral oil and a surfactant, wherein the adjuvant comprises up to 14,5 % Vol. of said surfactant
for use as a medicament.

2. The vaccine composition according to claim 1, wherein the surfactant is mannide oleate.

3. The vaccine composition according to claim 1-2, wherein one survivin peptide variant is consisting of: LMLGEFLKL (SEQ ID NO: 5).

4. The vaccine composition according to any of the claims 1-3, further comprising one or more peptides or peptide variants selected from the groups of ML-IAP, BCL-2, BCL-X, MCL-1 or TRAG-3 peptides or peptide variants thereof capable of binding a HLA class 1 molecule.

5. The vaccine composition according to any of the claims 1-4, further comprising one or more peptides or peptide variants selected from the groups of ML-IAP, BCL-X, MCL-1 or TRAG-3 peptides or peptide variants thereof capable of binding a HLA class 1 molecule.

6. The vaccine composition according to the claims 1-5, comprising a secondary active ingredient, selected from the group consisting of:
i. an anti-cancer medicament
ii. a chemotherapeutic agent and
iii. an angiogenesis inhibitor.

7. Use of a vaccine composition as **characterised by** any of the claims 1-6 for the manufacture of a medicament for the treatment of cancer.

8. The vaccine composition as **characterised by** any of the claims 1-6 for use in a method for treatment of cancer.

9. Use of a vaccine composition according to any of the claims 1-6, for the manufacture of a medicament for inhibition of angiogenesis.

10. The vaccine composition as **characterised by** any of the claims 1-6, for use in a method for inhibition of angiogenesis.

11. A kit in parts comprising;
iv. a vaccine composition according to any of the claims 1-6 and
v. a secondary medicament, wherein said secondary medicament is a chemotherapeutic agent or an angiogenesis inhibitor.

## Patentansprüche

1. Vakzin-Zusammensetzung, welche umfasst,
i. ein oder mehrere HLA-A2 beschränkte Survivin-Peptide, ausgewählt aus der Gruppe bestehend aus LLLGEFLKL (SEQ.ID.Nr. 4) und LMLGEFLKL (SEQ.ID.Nr. 5), und
ii. ein Adjuvans, formuliert für eine Wasser-in-Öl-Emulsion, die ein Mineralöl und ein oberflächenaktives Mittel umfasst, worin das Adjuvans bis zu 14,5 Vol.-% des oberflächenaktiven Mittels umfasst,
zur Verwendung als Medikament.

2. Vakzin-Zusammensetzung nach Anspruch 1, worin das oberflächenaktive Mittel Mannidoleat ist.

3. Vakzin-Zusammensetzung nach einem der Ansprüche 1 - 2, worin eine Survivin-Peptid-Variante besteht aus : LMLGEFLKL (SEQ.ID.Nr. 5).

4. Vakzin-Zusammensetzung nach einem der Ansprüche 1 - 3, welche weiter ein oder mehrere Peptide oder Peptid-Varianten umfasst ausgewählt aus den Gruppen von ML-IAP, BCL-2, BCL-X, MCL-1 oder TRAG-3 Peptiden oder Peptidvarianten davon, die an ein HLA-Molekül Klasse I binden können.

5. Vakzin-Zusammensetzung nach einem der Ansprüche 1 - 4, welche weiter ein oder mehrere Peptide oder Peptidvarianten umfasst ausgewählt aus den Gruppen bestehend aus ML-IAP, BCL-X, MCL-1 oder TRAG-3 Peptiden oder Peptidvarianten davon, die an ein HLA-Molekül Klasse I binden können.

6. Vakzin-Zusammensetzung nach einem der Ansprüche 1 - 5, welche weiter einen zweiten aktiven Inhaltsstoff umfasst, ausgewählt aus der Gruppe bestehend aus
i. einem anti-Krebs Medikament,
ii. einem chemotherapeutischen Medikament, und
iii. einem Angiogenese-Inhibitor.

7. Verwendung einer Vakzin-Zusammensetzung, gekennzeichnet nach einem der Ansprüche 1 - 6 zur Herstellung eines Medikaments zur Behandlung von Krebs

8. Vakzin-Zusammensetzung nach einem der Ansprüche 1 - 6 zur Verwendung in einem Verfahren zur Behandlung von Krebs.

9. Verwendung einer Vakzin-Zusammensetzung nach einem der Ansprüche 1 - 6 zur Herstellung eines Medikaments zur Inhibierung einer Angiogenese.

10. Vakzin-Zusammensetzung gekennzeichnet in nach einem der Ansprüche 1 - 6 zur Verwendung in einem Verfahren zur Inhibierung einer Angiogenese.

11. Kit in Teilen , welcher umfasst:
iv. eine Vakzin-Zusammensetzung nach einem der Ansprüche 1 - 6, und
v. ein zweites Medikament, worin das zweite Medikament ein chemotherapeutisches Mittel oder ein Angiogenese-Inhibitor ist.

## Revendications

1. Composition vaccinale comprenant :
i. un ou plusieurs peptides de la survivine restreints à HLA-A2 choisis dans le groupe constitué de LLLGEFLKL (SÉQ ID NO : 4) et LMLGEFLKL (SÉQ ID NO : 5), et
ii. un adjuvant formulé pour une émulsion eau dans l'huile comprenant une huile minérale et un tensioactif, l'adjuvant comprenant jusqu'à 14,5 % en volume dudit tensioactif
pour une utilisation en tant que médicament.

2. Composition vaccinale selon la revendication 1, dans laquelle le tensioactif est l'oléate de mannide.

3. Composition vaccinale selon les revendications 1 à 2, dans laquelle un variant de peptide de la survivine est constitué de : LMLGEFLKL (SÉQ ID NO : 5).

4. Composition vaccinale selon l'une quelconque des revendications 1 à 3, comprenant en outre un ou plusieurs peptides ou variants de peptides choisis dans les groupes des peptides ML-IAP, BCL-2, BCL-X, MCL-1 ou TRAG-3 ou des variants de ces peptides capables de lier une molécule HLA de classe 1.

5. Composition vaccinale selon l'une quelconque des revendications 1 à 4, comprenant en outre un ou plusieurs peptides ou variants de peptides choisis dans les groupes des peptides ML-IAP, BCL-X, MCL-1 ou TRAG-3 ou des variants de ces peptides capables de lier une molécule HLA de classe 1.

6. Composition vaccinale selon les revendications 1 à 5, comprenant un composant actif secondaire, choisi dans le groupe constitué de :
i. un médicament anticancéreux
ii. un agent chimiothérapeutique et
iii. un inhibiteur de l'angiogenèse.

7. Utilisation d'une composition vaccinale telle que **caractérisée par** l'une quelconque des revendications 1 à 6, pour la fabrication d'un médicament destiné au traitement du cancer.

8. Composition vaccinale telle que **caractérisée par** l'une quelconque des revendications 1 à 6, pour une utilisation dans un procédé de traitement du cancer.

9. Utilisation d'une composition vaccinale selon l'une quelconque des revendications 1 à 6, pour la fabrication d'un médicament destiné à l'inhibition de l'angiogenèse.

10. Composition vaccinale telle que **caractérisée par** l'une quelconque des revendications 1 à 6, pour une utilisation dans un procédé d'inhibition de l'angiogenèse.

11. Kits en parties comprenant :
iv. une composition vaccinale selon l'une quelconque des revendications 1 à 6 et
v. un médicament secondaire, ledit médicament secondaire étant un agent chimiothérapeutique ou un inhibiteur de l'angiogenèse.


### SEQUENCE LISTING

<110> SurvacApS
<120> Survivin peptide vaccine
<130> P984PC00
<160> 118
<170> PatentIn version 3.3
<210> 1
   <211 > 9
   <212> PRT
   < 213 > synthetic peptide
<220>
   <221> misc_feature
   <222> (1)..(9)
   <223> survivin101-109
<400> 1
   Phe Leu Lys Leu Asp Arg Glu Arg Ala
   1 5
<210> 2
   <211> 10
   <212> PRT
   <213> synthetic peptide
<220>
   <221> MISC_FEATURE
   <222> (1)..(10)
   <223> Survivin5-14
<400> 2
   Thr Leu Pro Pro Ala Trp Gln Pro Phe Leu
   1 5 10
<210> 3
   <211> 10
   <212> PRT
   <213> synthetic
<220>
   <221> misc_feature
   <222> (1)..(10)
   <223> survivin95-104
<400> 3
   Glu Leu Thr Leu Gly Glu Phe Leu Lys Leu
   1 5 10
<210> 4
   <211> 9
   <212> PRT
   <213> synthetic peptide
<220>
   <221> misc_feature
   <222> (1)..(9)
   <223> survivin96-104L2
<400> 4
   Leu Leu Leu Gly Glu Phe Leu Lys Leu
   1 5
<210> 5
   <211 > 9
   <212> PRT
   < 213 > synthetic peptide
<220>
   <221> misc_feature
   <222> (1)..(9)
   <223> survivin96-104M2
<400> 5
   Leu Met Leu Gly Glu Phe Leu Lys Leu
   1 5
<210> 6
   <211 > 9
   <212> PRT
   <213> synthetic peptide
<220>
   <221> misc_feature
   <222> (1)..(9)
   <223> survivin46-54
<400> 6
   Cys Pro Thr Glu Asn Glu Pro Asp Leu
   1 5
<210> 7
   <211> 9
   <212> PRT
   <213> synthetic peptide
<220>
   <221> misc_feature
   <222> (1)..(9)
   <223> SURVIVIN51-59
<400> 7
   Glu Pro Asp Leu Ala Gln Cys Phe Phe
   1 5
<210> 8
   <211> 9
   <212> PRT
   <213> synthetic peptide
<220>
   <221> misc_feature
   <222> (1)..(9)
   <223> SURVIVIN46-54Y9
<400> 8
   Cys Pro Thr Glu Asn Glu Pro Asp Tyr
   1 5
<210> 9
   <211> 9
   <212> PRT
   <213> Synthetic peptide
<220>
   <221> misc_feature
   <222> (1)..(9)
   <223> SURVIVIN51-59Y9
<400> 9
   Glu Pro Asp Leu Ala Gln Cys Phe Tyr
   1 5
<210> 10
   <211> 9
   <212> PRT
   <213> synthetic peptide
<220>
   <221> misc_feature
   <222> (1)..(9)
   <223> SURVIVIN6-14
<400> 10
   Leu Pro Pro Ala Trp Gln Pro Phe Leu
   1 5
<210> 11
   <211> 9
   <212> PRT
   < 213 > synthetic peptide
<220>
   <221> misc_feature
   <222> (1)..(9)
   <223> SURVIVIN11-19
<400> 11
   Gln Pro Phe Leu Lys Asp His Arg Ile
   1 5
<210> 12
   <211 > 10
   <212> PRT
   <213> synthetic peptide
<220>
   <221> misc_feature
   <222> (1)..(10)
   <223> SURVIVIN34-43
<400> 12
   Thr Pro Glu Arg Met Ala Glu Ala Gly Phe
   1 5 10
<210> 13
   <211> 9
   <212> PRT
   <213> synthetic peptide
<220>
   <221> misc_feature
   <222> (1)..(9)
   <223> SURVIVIN6-14A1
<400> 13
   Ala Pro Pro Ala Trp Gln Pro Phe Leu
   1 5
<210> 14
   <211> 9
   <212> PRT
   <213> synthetic peptide
<220>
   <221> misc_feature
   <222> (1)..(9)
   <223> SURVIVIN6-14R1
<400> 14
   Arg Pro Pro Ala Trp Gln Pro Phe Leu
   1 5
<210> 15
   <211 > 10
   <212> PRT
   <213> synthetic peptide
<220>
   <221> misc_feature
   <222> (1)..(10)
   <223> SURVIVIN92-101
<400> 15
   Gln Phe Glu Glu Leu Thr Leu Gly Glu Phe
   1 5 10
<210> 16
   <211> 9
   <212> PRT
   <213> Synthetic peptide
<220>
   <221> misc_feature
   <222> (1)..(9)
   <223> SURVIVIN93-101T2
<400> 16
   Phe Thr Glu Leu Thr Leu Gly Glu Phe
   1 5
<210> 17
   <211> 9
   <212> PRT
   < 213 > Synthetic peptide
<220>
   <221> misc_feature
   <222> (1)..(9)
   <223> SURVIVIN38-46Y9
<400> 17
   Met Ala Glu Ala Gly Phe Ile His Tyr
   1 5
<210> 18
   <211> 10
   <212> PRT
   < 213 > Synthetic peptide
<220>
   <221> misc_feature
   <222> (1)..(10)
   <223> SURVIVIN47-56Y10
<400> 18
   Pro Thr Glu Asn Glu Pro Asp Leu Ala Tyr
   1 5 10
<210> 19
   <211> 10
   <212> PRT
   <213> synthetic peptide
<220>
   <221> misc_feature
   <222> (1)..(10)
   <223> SURVIVIN53-62
<400> 19
   Asp Leu Ala Gln Cys Phe Phe Cys Phe Lys
   1 5 10
<210> 20
   <211> 10
   <212> PRT
   <213> Synthetic peptide
<220>
   <221> misc_feature
   <222> (1)..(10)
   <223> Survivin18-27K10
<400> 20
   Arg Ile Ser Thr Phe Lys Asn Trp Pro Lys
   1 5 10
<210> 21
   <211 > 11
   <212> PRT
   <213> synthetic peptide
<220>
   <221> misc_feature
   <222> (1)..(11)
   <223> SURVIVIN18-28
<400> 21
   Arg Ile Ser Thr Phe Lys Asn Trp Pro Phe Leu
   1 5 10
<210> 22
   <211 > 429
   <212> DNA
   <213> homo sapiens
<220>
   <221> CDS
   <222> (1)..(426)
<400> 22
   atg ggt gcc ccg acg ttg ccc cct gcc tgg cag ccc ttt ctc aag gac      48
   Met Gly Ala Pro Thr Leu Pro Pro Ala Trp Gln Pro Phe Leu Lys Asp
   1               5                   10                  15
   cac cgc atc tct aca ttc aag aac tgg ccc ttc ttg gag ggc tgc gcc      96
   His Arg Ile Ser Thr Phe Lys Asn Trp Pro Phe Leu Glu Gly Cys Ala
               20                  25                  30
   tgc acc ccg gag cgg atg gcc gag gct ggc ttc atc cac tgc ccc act      144
   Cys Thr Pro Glu Arg Met Ala Glu Ala Gly Phe Ile His Cys Pro Thr
           35                  40                  45
   gag aac gag cca gac ttg gcc cag tgt ttc ttc tgc ttc aag gag ctg      192
   Glu Asn Glu Pro Asp Leu Ala Gln Cys Phe Phe Cys Phe Lys Glu Leu
       50                  55                  60
   gaa ggc tgg gag cca gat gac gac ccc ata gag gaa cat aaa aag cat      240
   Glu Gly Trp Glu Pro Asp Asp Asp Pro Ile Glu Glu His Lys Lys His
   65                  70                  75                  80
   tcg tcc ggt tgc gct ttc ctt tct gtc aag aag cag ttt gaa gaa tta      288
   Ser Ser Gly Cys Ala Phe Leu Ser Val Lys Lys Gln Phe Glu Glu Leu
                   85                  90                  95
   acc ctt ggt gaa ttt ttg aaa ctg gac aga gaa aga gcc aag aac aaa      336
   Thr Leu Gly Glu Phe Leu Lys Leu Asp Arg Glu Arg Ala Lys Asn Lys
               100                 105                 110
   att gca aag gaa acc aac aat aag aag aaa gaa ttt gag gaa act gcg      384
   Ile Ala Lys Glu Thr Asn Asn Lys Lys Lys Glu Phe Glu Glu Thr Ala
           115                 120                 125
   aag aaa gtg cgc cgt gcc atc gag cag ctg gct gcc atg gat tga          429
   Lys Lys Val Arg Arg Ala Ile Glu Gln Leu Ala Ala Met Asp
       130                 135                 140
<210> 23
   <211 > 142
   <212> PRT
   <213> homo sapiens
<400> 23
   Met Gly Ala Pro Thr Leu Pro Pro Ala Trp Gln Pro Phe Leu Lys Asp
   1 5 10 15
   His Arg Ile Ser Thr Phe Lys Asn Trp Pro Phe Leu Glu Gly Cys Ala
   20 25 30
   Cys Thr Pro Glu Arg Met Ala Glu Ala Gly Phe Ile His Cys Pro Thr
   35 40 45
   Glu Asn Glu Pro Asp Leu Ala Gln Cys Phe Phe Cys Phe Lys Glu Leu
   50 55 60
   Glu Gly Trp Glu Pro Asp Asp Asp Pro Ile Glu Glu His Lys Lys His
   65 70 75 80
   Ser Ser Gly Cys Ala Phe Leu Ser Val Lys Lys Gln Phe Glu Glu Leu
   85 90 95
   Thr Leu Gly Glu Phe Leu Lys Leu Asp Arg Glu Arg Ala Lys Asn Lys
   100 105 110
   Ile Ala Lys Glu Thr Asn Asn Lys Lys Lys Glu Phe Glu Glu Thr Ala
   115 120 125
   Lys Lys Val Arg Arg Ala Ile Glu Gln Leu Ala Ala Met Asp
   130 135 140
<210> 24
   <211 > 10
   <212> PRT
   <213> Homo sapiens
<400> 24
   Arg Leu Gln Glu Glu Arg Thr Cys Lys Val
   1 5 10
<210> 25
   <211 > 10
   <212> PRT
   <213> Homo sapiens
<400> 25
   Gln Leu Cys Pro Ile Cys Arg Ala Pro Val
   1 5 10
<210> 26
   <211> 10
   <212> PRT
   <213> Homo sapiens
<400> 26
   Arg Leu Ala Ser Phe Tyr Asp Trp Pro Leu
   1 5 10
<210> 27
   <211> 10
   <212> PRT
   <213> Homo sapiens
<400> 27
   Leu Leu Arg Ser Lys Gly Arg Asp Phe Val
   1 5 10
<210> 28
   <211> 10
   <212> PRT
   <213> Homo sapiens
<400> 28
   Val Leu Glu Pro Pro Gly Ala Arg Asp Val
   1 5 10
<210> 29
   <211> 10
   <212> PRT
   <213> Homo sapiens
<400> 29
   Pro Leu Thr Ala Glu Val Pro Pro Glu Leu
   1 5 10
<210> 30
   <211> 9
   <212> PRT
   <213> Homo sapiens
<400> 30
   Ser Leu Gly Ser Pro Val Leu Gly Leu
   1 5
<210> 31
   <211> 9
   <212> PRT
   <213> Homo sapiens
<400> 31
   Gln Ile Leu Gly Gln Leu Arg Pro Leu
   1 5
<210> 32
   <211> 9
   <212> PRT
   <213> Homo sapiens
<400> 32
   Leu Thr Ala Glu Val Pro Pro Glu Leu
   1 5
<210> 33
   <211> 9
   <212> PRT
   <213> Homo sapiens
<400> 33
   Gly Met Gly Ser Glu Glu Leu Arg Leu
   1 5
<210> 34
   <211> 9
   <212> PRT
   <213> Homo sapiens
<400> 34
   Glu Leu Pro Thr Pro Arg Arg Glu Val
   1 5
<210> 35
   <211> 10
   <212> PRT
   < 213 > synthetic sequence
<400> 35
   Asn Ile Ala Leu Trp Met Thr Glu Tyr Leu
   1 5 10
<210> 36
   <211> 10
   <212> PRT
   <213> synthetic sequence
<400> 36
   Tyr Leu Asn Arg His Leu His Thr Trp Ile
   1 5 10
<210> 37
   <211> 10
   <212> PRT
   <213> synthetic sequence
<400> 37
   Pro Leu Phe Asp Phe Ser Trp Leu Ser Leu
   1 5 10
<210> 38
   <211> 10
   <212> PRT
   <213> synthetic sequecne
<400> 38
   Trp Leu Ser Leu Lys Thr Leu Leu Ser Leu
   1 5 10
<210> 39
   <211 > 9
   <212> PRT
   < 213 > synthetic sequence
<400> 39
   Lys Thr Leu Leu Ser Leu Ala Leu Val
   1 5
<210> 40
   <211> 10
   <212> PRT
   <213> synthetic sequnce
<400> 40
   Ala Ala Ala Gly Pro Ala Leu Ser Pro Val
   1 5 10
<210> 41
   <211> 9
   <212> PRT
   <213> synthetic peptide
<220>
   <221> MISC_FEATURE
   <222> (1)..(9)
   <223> Sur20-28
<400> 41
   Ser Thr Phe Lys Asn Trp Pro Phe Leu
   1 5
<210> 42
   <211> 10
   <212> PRT
   <213> synthetic peptide
<220>
   <221> MISC_FEATURE
   <222> (1)..(10)
   <223> Sur115Y10
<400> 42
   Lys Glu Thr Asn Asn Lys Lys Lys Glu Tyr
   1 5 10
<210> 43
   <211> 9
   <212> PRT
   <213> synthetic peptide
<220>
   <221> MISC_FEATURE
   <222> (1)..(9)
   <223> Sur36-44
<400> 43
   Glu Arg Met Ala Glu Ala Gly Phe Ile
   1 5
<210> 44
   <211> 9
   <212> PRT
   < 213 > synthetic peptide
<220>
   <221> MISC_FEATURE
   <222> (1)..(9)
   <223> Sur133-141
<400> 44
   Arg Ala Ile Glu Gln Leu Ala Ala Met
   1 5
<210> 45
   <211> 10
   <212> PRT
   <213> synthetic peptide
<400> 45
   Asp Val Ala Gln Cys Phe Phe Cys Phe Lys
   1 5 10
<210> 46
   <211 > 10
   <212> PRT
   <213> Synthetic peptide
<400> 46
   Asp Phe Ala Gln Cys Phe Phe Cys Phe Lys
   1 5 10
<210> 47
   <211> 10
   <212> PRT
   <213 > SYNTHETIC PEPTIDE
<400> 47
   Asp Ile Ala Gln Cys Phe Phe Cys Phe Lys
   1 5 10
<210> 48
   <211 > 9
   <212> PRT
   <213> synthetic peptide
<400> 48
   Ser Tyr Phe Lys Asn Trp Pro Phe Leu
   1 5
<210> 49
   <211 > 9
   <212> PRT
   < 213 > synthetic peptide
<400> 49
   Thr Ala Lys Lys Val Arg Arg Ala Ile
   1 5
<210> 50
   <211 > 9
   <212> PRT
   < 213 > synthetic peptide
<400> 50
   Arg Pro Ile Glu Gln Leu Ala Ala Met
   1 5
<210> 51
   <211> 9
   <212> PRT
   <213> synthetic peptide
<400> 51
   Thr Pro Lys Lys Val Arg Arg Ala Ile
   1 5
<210> 52
   <211 > 9
   <212> PRT
   <213> synthetic peptide
<400> 52
   Glu Arg Ala Lys Asn Lys Ile Ala Lys
   1 5
<210> 53
   <211 > 9
   <212> PRT
   <213> synthetic peptide
<400> 53
   Asp Arg Glu Arg Ala Lys Asn Lys Ile
   1 5
<210> 54
   <211 > 9
   <212> PRT
   <213> synthetic peptide
<400> 54
   Lys Glu Phe Glu Glu Thr Ala Lys Lys
   1 5
<210> 55
   <211 > 9
   <212> PRT
   <213> synthetic peptide
<400> 55
   Glu Arg Met Ala Glu Ala Gly Phe Leu
   1 5
<210> 56
   <211 > 8
   <212> PRT
   < 213 > synthetic peptide
<400> 56
   Glu Arg Ala Glu Ala Gly Phe Phe
   1 5
<210> 57
   <211> 9
   <212> PRT
   < 213 > synthetic peptide
<400> 57
   Glu Arg Met Ala Glu Ala Gly Phe Arg
   1 5
<210> 58
   <211 > 9
   <212> PRT
   < 213 > synthetic peptide
<400> 58
   Glu Arg Met Ala Glu Ala Gly Phe Lys
   1 5
<210> 59
   <211> 9
   <212> PRT
   <213> synthetic peptide
<400> 59
   Lys Arg Phe Glu Glu Thr Ala Lys Lys
   1 5
<210> 60
   <211 > 10
   <212> PRT
   < 213 > synthetic peptide
<400> 60
   Lys Glu Thr Asn Asn Lys Lys Lys Glu Phe
   1 5 10
<210> 61
   <211 > 9
   <212> PRT
   <213> synthetic peptide
<400> 61
   Glu Glu Leu Thr Leu Gly Glu Phe Leu
   1 5
<210> 62
   <211> 9
   <212> PRT
   <213> synthetic peptide
<400> 62
   Glu Glu Leu Thr Leu Gly Glu Phe Tyr
   1 5
<210> 63
   <211> 10
   <212> PRT
   < 213 > synthetic peptide
<400> 63
   Ile Ser Thr Phe Lys Asn Trp Pro Phe Leu
   1 5 10
<210> 64
   <211> 10
   <212> PRT
   <213> synthetic peptide
<400> 64
   Ile Ser Lys Phe Lys Asn Trp Pro Phe Leu
   1 5 10
<210> 65
   <211> 10
   <212> PRT
   <213> synthetic peptide
<400> 65
   Leu Ser Val Lys Lys Gln Phe Glu Glu Leu
   1 5 10
<210> 66
   <211 > 10
   <212> PRT
   <213> synthetic peptide
<400> 66
   Leu Ser Lys Lys Lys Gln Phe Glu Glu Leu
   1 5 10
<210> 67
   <211> 10
   <212> PRT
   <213> synthetic peptide
<400> 67
   Arg Ala Lys Asn Lys Ile Ala Lys Glu Thr
   1 5 10
<210> 68
   <211> 10
   <212> PRT
   < 213 > synthetic peptide
<400> 68
   Arg Ala Lys Asn Lys Ile Ala Lys Glu Leu
   1 5 10
<210> 69
   <211 > 10
   <212> PRT
   < 213 > synthetic peptide
<400> 69
   Asn Asn Lys Lys Lys Glu Phe Glu Glu Thr
   1 5 10
<210> 70
   <211> 10
   <212> PRT
   <213> synthetic peptide
<400> 70
   Asn Asn Lys Lys Lys Glu Phe Glu Glu Leu
   1 5 10
<210> 71
   <211 > 9
   <212> PRT
   < 213 > synthetic peptide
<400> 71
   Gln Pro Lys Leu Lys Asp His Arg Ile
   1 5
<210> 72
   <211 > 9
   <212> PRT
   <213> synthetic peptide
<400> 72
   Phe Leu Lys Asp His Arg Ile Ser Thr
   1 5
<210> 73
   <211 > 9
   <212> PRT
   < 213 > synthetic peptide
<400> 73
   Phe Leu Lys Asp Lys Arg Ile Ser Thr
   1 5
<210> 74
   <211 > 9
   <212> PRT
   < 213 > synthetic peptide
<400> 74
   Phe Leu Lys Asp His Arg Ile Ser Leu
   1 5
<210> 75
   <211 > 9
   <212> PRT
   < 213 > synthetic peptide
<400> 75
   Ala Phe Leu Ser Val Lys Lys Gln Phe
   1 5
<210> 76
   <211> 9
   <212> PRT
   <213> synthetic peptide
<400> 76
   Ala Phe Lys Ser Val Lys Lys Gln Phe
   1 5
<210> 77
   <211 > 9
   <212> PRT
   <213> synthetic peptide
<400> 77
   Ala Phe Leu Ser Lys Lys Lys Gln Phe
   1 5
<210> 78
   <211 > 9
   <212> PRT
   <213> synthetic peptide
<400> 78
   Ala Phe Leu Ser Val Lys Lys Gln Leu
   1 5
<210> 79
   <211 > 9
   <212> PRT
   <213> synthetic peptide
<400> 79
   Phe Leu Ser Val Lys Lys Gln Phe Glu
   1 5
<210> 80
   <211 > 9
   <212> PRT
   <213> synthetic peptide
<400> 80
   Phe Leu Ser Val Lys Lys Gln Phe Leu
   1 5
<210> 81
   <211 > 9
   <212> PRT
   <213> synthetic peptide
<400> 81
   Phe Leu Lys Val Lys Lys Gln Phe Glu
   1 5
<210> 82
   <211 > 9
   <212> PRT
   <213> synthetic peptide
<400> 82
   Ser Val Lys Lys Gln Phe Glu Glu Leu
   1 5
<210> 83
   <211 > 9
   <212> PRT
   <213> synthetic peptide
<400> 83
   Ser Val Lys Lys Lys Phe Glu Glu Leu
   1 5
<210> 84
   <211 > 9
   <212> PRT
   <213> synthetic peptide
<400> 84
   Phe Leu Lys Leu Lys Arg Glu Arg Ala
   1 5
<210> 85
   <211 > 9
   <212> PRT
   <213> synthetic peptide
<400> 85
   Phe Leu Lys Leu Asp Arg Glu Arg Leu
   1 5
<210> 86
   <211> 9
   <212> PRT
   <213> synthetic peptide
<400> 86
   Thr Ala Lys Lys Lys Arg Arg Ala Ile
   1 5
<210> 87
   <211 > 9
   <212> PRT
   <213> synthetic peptide
<400> 87
   Thr Ala Lys Lys Val Arg Arg Ala Leu
   1 5
<210> 88
   <211 > 9
   <212> PRT
   <213> synthetic peptide
<400> 88
   Thr Leu Pro Pro Ala Trp Gln Pro Phe
   1 5
<210> 89
   <211 > 9
   <212> PRT
   <213> synthetic peptide
<400> 89
   Thr Leu Pro Pro Ala Trp Gln Pro Tyr
   1 5
<210> 90
   <211> 10
   <212> PRT
   <213> synthetic peptide
<400> 90
   Trp Gln Pro Phe Leu Lys Asp His Arg Ile
   1 5 10
<210> 91
   <211> 10
   <212> PRT
   <213> synthetic peptide
<400> 91
   Trp Gln Pro Phe Leu Lys Asp His Arg Tyr
   1 5 10
<210> 92
   <211> 10
   <212> PRT
   < 213 > synthetic peptide
<400> 92
   Phe Leu Lys Asp His Arg Ile Ser Thr Phe
   1 5 10
<210> 93
   <211> 10
   <212> PRT
   < 213 > synthetic peptide
<400> 93
   Phe Leu Lys Asp His Arg Ile Ser Thr Tyr
   1 5 10
<210> 94
   <211 > 10
   <212> PRT
   < 213 > synthetic peptide
<400> 94
   Arg Ile Ser Thr Phe Lys Asn Trp Pro Phe
   1 5 10
<210> 95
   <211 > 10
   <212> PRT
   <213> synthetic peptide
<400> 95
   Arg Leu Ser Thr Phe Lys Asn Trp Pro Phe
   1 5 10
<210> 96
   <211 > 9
   <212> PRT
   <213> synthetic peptide
<400> 96
   Asp Leu Ala Gln Cys Phe Phe Cys Phe
   1 5
<210> 97
   <211 > 9
   <212> PRT
   <213> synthetic peptide
<400> 97
   Asp Leu Ala Gln Cys Phe Phe Cys Tyr
   1 5
<210> 98
   <211 > 9
   <212> PRT
   <213> synthetic peptide
<400> 98
   Ile Ser Thr Phe Lys Asn Trp Pro Phe
   1 5
<210> 99
   <211 > 9
   <212> PRT
   < 213 > synthetic peptide
<400> 99
   Ile Gln Thr Phe Lys Asn Trp Pro Phe
   1 5
<210> 100
   <211> 9
   <212> PRT
   <213> synthetic peptide
<400> 100
   Ile Leu Thr Phe Lys Asn Trp Pro Phe
   1 5
<210> 101
   <211> 9
   <212> PRT
   < 213 > synthetic peptide
<400> 101
   Arg Met Ala Glu Ala Gly Phe Ile Tyr
   1 5
<210> 102
   <211> 9
   <212> PRT
   <213> synthetic peptide
<400> 102
   Arg Met Ala Glu Ala Gly Phe Ile Phe
   1 5
<210> 103
   <211> 9
   <212> PRT
   <213> synthetic peptide
<400> 103
   Arg Leu Ala Glu Ala Gly Phe Ile Tyr
   1 5
<210> 104
   <211 > 9
   <212> PRT
   <213> synthetic peptide
<400> 104
   Lys Lys His Ser Ser Gly Cys Ala Phe
   1 5
<210> 105
   <211> 9
   <212> PRT
   < 213 > synthetic peptide
<400> 105
   Lys Gln His Ser Ser Gly Cys Ala Phe
   1 5
<210> 106
   <211 > 9
   <212> PRT
   <213> synthetic peptide
<400> 106
   Lys Leu His Ser Ser Gly Cys Ala Phe
   1 5
<210> 107
   <211> 9
   <212> PRT
   < 213 > synthetic peptide
<400> 107
   Arg Ala Ile Glu Gln Leu Ala Ala Tyr
   1 5
<210> 108
   <211 > 9
   <212> PRT
   < 213 > synthetic peptide
< 400 > 108
   Arg Leu Ile Glu Gln Leu Ala Ala Met
   1 5
<210> 109
   <211 > 10
   <212> PRT
   <213> synthetic peptide
<400> 109
   Pro Thr Leu Pro Pro Ala Trp Gln Pro Phe
   1 5 10
<210> 110
   <211> 11
   <212> PRT
   <213> synthetic peptide
<400> 110
   Cys Thr Pro Glu Arg Met Ala Glu Ala Gly Phe
   1 5 10
<210> 111
   <211> 9
   <212> PRT
   <213> synthetic peptide
<400> 111
   Glu Thr Asn Asn Lys Lys Lys Glu Phe
   1 5
<210> 112
   <211 > 9
   <212> PRT
   < 213 > synthetic peptide
<400> 112
   Gly Ala Pro Thr Leu Pro Pro Ala Trp
   1 5
<210> 113
   <211> 10
   <212> PRT
   <213> synthetic peptide
<400> 113
   Cys Ala Phe Leu Ser Val Lys Lys Gln Phe
   1 5 10
<210> 114
   <211 > 10
   <212> PRT
   <213> synthetic peptide
<400> 114
   Thr Pro Glu Arg Met Ala Glu Ala Gly Phe
   1 5 10
<210> 115
   <211 > 9
   <212> PRT
   < 213 > synthetic peptide
<400> 115
   Cys Ala Cys Thr Pro Glu Arg Met Ala
   1 5
<210> 116
   <211> 9
   <212> PRT
   <213> synthetic peptide
<400> 116
   Cys Thr Pro Glu Arg Met Ala Glu Ala
   1 5
<210> 197
   <211> 9
   <212> PRT
   <213> synthetic peptide
<400> 117
<210> 118
   <211> 10
   <212> PRT
   <213> synthetic peptide
<400> 118
   Trp Pro Phe Leu Glu Gly Cys Ala Cys Thr
   1 5 10